# EUROPÄISCHE PATENTSCHRIFT

(11) **EP 0 727 417 B1**
(45) Veröffentlichungstag und Bekanntmachung des Hinweises auf die Patenterteilung: **07.03.2001**
(21) Anmeldenummer: 96101977.5
(22) Anmeldetag: 12.02.1996
(51) Int. Cl.: C07C 311/58, C07C 335/42, A61K 31/64

(54) **Substituierte Benzolsulfonylharnstoffe und -thioharnstoffe, Verfahren zu ihrer Herstellung und Verwendung pharmazeutischer Präparate auf Basis dieser Verbindungen sowie sie enthaltende Heilmittel**
Substituted benzenesulfonylureas and -thioureas, process for their preparation and the use of pharmaceutical preparations based on these compounds as well as medicaments containing them
Benzènesulfonylurées et -thiourées substituées, procédé pour leur préparation et l'utilisation de préparations pharmaceutiques à base de ces composés ainsi que médicaments les contenant

(30) Priorität: 17.02.1995 DE 19505398; 23.06.1995 DE 19522920
(43) Veröffentlichungstag der Anmeldung: 21.08.1996
(73) Patentinhaber: HOECHST AKTIENGESELLSCHAFT, 65929 Frankfurt am Main (DE)
(72) Erfinder: Englert, Heinrich, Dr, D-65719 Hofheim (DE); Gerlach, Uwe, Dr., D-65795 Hattersheim (DE); Mania, Dieter, Dr., D-61462 Königstein (DE); Gögelein, Heinz, Dr., D-60259 Frankfurt (DE); Kaiser, Joachim, Dr., D-60431 Frankfurt (DE)

(56) Entgegenhaltungen:
- EP-A- 0 612 724
- DE-A- 1 939 895
- DE-B- 1 198 354

## Beschreibung

Die Erfindung betrifft substituierte Benzolsulfonylharnstoffe und -thioharnstoffe I 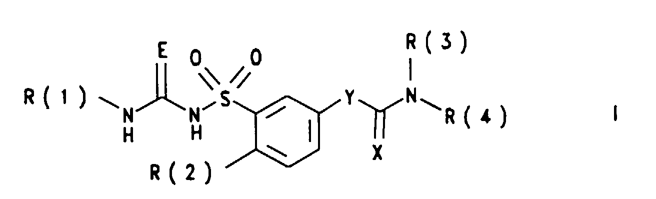 worin bedeuten:
- R(1): Wasserstoff, Alkyl mit 1, 2, 3, 4, 5, 6 oder 7 C-Atomen, Cycloalkyl mit 3, 4, 5, 6 oder 7 C-Atomen;
- R(2): Wasserstoff, Alkyl mit 1, 2, 3, 4, 5 oder 6 C-Atomen, Cycloalkyl mit 3, 4, 5 oder 6 C-Atomen oder eine (C₁-C₈)-Kette, in der n C-Atome durch Heteroatome, z.B. O, N, S, ersetzt sind ( mit n = 1 - 4), F, Cl;
- R(3): H, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. O, N, S, ersetzt sind (n = 1, 2, 3 oder 4);
- R(4): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. 0, N, S, ersetzt sind (n = 1, 2, 3 oder 4);
oder
- R(3) und R(4): gemeinsam einen (CH₂)₂₋₈-Ring bilden, in dem eine oder mehrere der CH₂-Gruppen durch Heteroatome ersetzt sein können;
- E: Sauerstoff oder Schwefel;
- x: Sauerstoff oder Schwefel;
- Y: [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
- m: 1, 2, 3 oder 4;
sowie deren pharmazeutisch verträgliche Salze.

Der Begriff Alkyl beschreibt, sofern nicht anders angegeben, geradkettige oder verzweigte gesättigte Kohlenwasserstoffreste. Der Cycloalkylrest kann zusätzlich einen Alkylsubstituenten tragen. Als Halogensubstituent sind die Elemente Fluor, Chlor, Brom und lod einsetzbar. Weiterhin können Verbindungen mit Chiralitätszentren in den Alkylketten Y, R(3) und R(4) auftreten. In diesem Fall gehören sowohl die einzelnen Antipoden für sich, als auch eine Mischung der beiden Enantiomere in unterschiedlichen Verhältnissen, sowie die dazugehörigen Mesoverbindungen oder Mischungen aus Mesoverbindungen, den Enantiomeren oder Diastereomeren zur Erfindung.

Ähnliche Sulfonylharnstoffe sind aus der Deutschen Offenlegungsschrift 1 198 354 bekannt. Darin werden die blutzuckersenkenden Wirkungen der Sulfonylharnstoffe beschrieben. Als Prototyp solcher blutzuckersenkenden Sulfonylharnstoffe gilt das Glibenclamid, das als Mittel zur Behandlung des Diabetes mellitus therapeutisch verwendet wird und in der Wissenschaft ein vielbeachtetes Werkzeug zur Erforschung sogenannter ATP-sensitiver Kaliumkanäle dient. Neben seiner blutzuckersenkenden Wirkung besitzt das Glibenclamid noch andere Wirkungen, die bislang therapeutisch noch nicht eingesetzt werden können, die aber allesamt auf Blockade eben dieser ATP-sensitiven Kalium-Kanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich, da sie den Zustand des Patienten weiter verschlechtern kann.

Bestimmte Benzolsulfonylharnstoffe und -thioharnstoffe, die eine verminderte blutzuckersenkende Wirkung aufweisen, werden in der EP-A-612 724 beschrieben. Diese Verbindungen enthalten eine Benzoylaminoalkyl-Gruppierung. Aufgabe der vorliegenden Erfindung war es, weitere Verbindungen zu synthetisieren, die eine gleich gute Herzwirkung wie Glibenclamid aufweisen, aber den Blutzucker in herzwirksamen Dosen oder Konzentrationen nicht oder deutlich geringer beeinflussen als Glibenclamid.

Als Versuchstiere zum Nachweis solcher Wirkungen eignen sich zum Beispiel Mäuse, Ratten, Meerschweinchen, Kaninchen, Hunde, Affen oder Schweine.

Die Verbindungen I dienen als Arzneimittelwirkstoffe in der Human- und Veterinärmedizin. Ferner können sie als Zwischenprodukte zur Herstellung weiterer Arzneimittelwirkstoffe verwendet werden.

Bevorzugt sind die Verbindungen I, worin bedeuten:
- R(1): Wasserstoff, Alkyl mit 1, 2, 3, 4, 5, 6 oder 7 C-Atomen, Cycloalkyl mit 3, 4, 5, 6 oder 7 C-Atomen;
- R(2): Wasserstoff, F, Cl, Alkyl mit 1, 2, 3, 4, 5 oder 6 C-Atomen, Cycloalkyl mit 3, 4, 5 oder 6 C-Atomen oder eine (C₂-C₇)-Kette, in der 1, 2, 3 oder 4 CH₂-Gruppen durch Heteroatome 0, N, oder S ersetzt sind;
- R(3) und R(4): gleich oder verschieden Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der 1, 2, 3 oder 4 CH₂-Gruppen durch Heteroatome O, S oder NH ersetzt sind;
oder
- R(3) und R(4): gemeinsam (CH₂)₂₋₈, worin eine der CH₂-Gruppen durch ein Heteroatom O, S oder NH ersetzt sein kann;
- E: Sauerstoff oder Schwefel;
- x: Sauerstoff oder Schwefel;
- Y: [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
- m: 1, 2, 3 oder 4;
sowie deren pharmazeutisch verträgliche Salze.

Besonders bevorzugt sind Verbindungen der Formel I, worin bedeuten:
- R(1): Wasserstoff, Alkyl mit 1, 2, 3 oder 4 C-Atomen, Cycloalkyl mit 3, 4, 5, 6 oder 7 C-Atomen;
- R(2): F, Cl, Alkyl mit 1, 2, 3 oder 4 C-Atomen oder eine (C₂-C₇)-Kette, worin 1, 2, 3 oder 4 CH₂-Gruppen durch Heteroatome O, S oder NH ersetzt sind;
- R(3) und R(4): gleich oder verschieden Wasserstoff, Alkyl mit 1, 2, 3, 4, 5, 6, 7, 8, 9 oder 10 C-Atomen, Cycloalkyl mit 3, 4, 5 oder 6 C-Atomen oder eine (C₁-C₁₀)-Kette, worin 1, 2, 3 oder 4 CH₂-Gruppen durch Heteroatome O, S, NH ersetzt sind;
oder
- R(3) und R(4): gemeinsam (CH₂)₂₋₇, worin eine der CH₂-Gruppen durch Heteroatome O, S, NH ersetzt sein kann;
- E: Sauerstoff oder Schwefel;
- x: Sauerstoff oder Schwefel;
- Y: [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
- m: 1, 2, 3 oder 4;
sowie deren pharmazeutisch verträgliche Salze.

Bevorzugt sind auch Verbindungen der Formel I, worin bedeuten:
- R(1): Wasserstoff, Alkyl mit 1, 2, 3, 4, 5, 6 oder 7 C-Atomen, Cycloalkyl mit 3, 4, 5, 6 oder 7 C-Atomen;
- R(2): Alkoxy mit 1, 2, 3, 4, 5 oder 6 C-Atomen oder Mercaptoalkyl mit 1, 2, 3, 4, 5 oder 6 C-Atomen;
- R(3): H, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. O, N, S, ersetzt sind (n = 1, 2, 3 oder 4);
- R(4): Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. O, N, S, ersetzt sind (n = 1, 2, 3 oder 4);
oder
- R(3) und R(4): gemeinsam einen (CH₂)₂₋₈-Ring bilden, in dem eine oder mehrere der CH₂-Gruppen durch Heteroatome ersetzt sein können;
- E: Sauerstoff oder Schwefel;
- X: Sauerstoff oder Schwefel;
- Y: [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
m 1, 2, 3 oder 4;
sowie deren pharmazeutisch verträgliche Salze.

Ganz besonders bevorzugt sind die Verbindungen I, worin bedeuten:
- R(1): Wasserstoff, Alkyl mit 1, 2, 3 oder 4 C-Atomen, Cycloalkyl mit 3 oder 4 C-Atomen;
- R(2): Methoxy oder Ethoxy;
- R(3) und R(4): gleich oder verschieden Wasserstoff, Alkyl mit 3, 4, 5, 6, 7 oder 8 C-Atomen, Cycloalkyl mit 3, 4, 5 oder 6 C-Atomen oder eine (C₃₋₈H₇₋₁₇)-Gruppe, worin 1, 2, 3 oder 4 CH₂-Gruppen durch Heteroatome O, S, NH ersetzt sind;
oder
- R(3) und R(4): gemeinsam eine (CH₂)₂₋₈-Gruppe, worin eine der CH₂-Gruppen durch Heteroatome O, S, NH ersetzt sein kann;
- E: Schwefel oder Sauerstoff;
- x: Sauerstoff;
- Y: [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
m 1 oder 2;
sowie deren pharmazeutisch verträgliche Salze.

Ebenfalls ganz besonders bevorzugt sind die Verbindungen I, worin bedeuten:
- R(1): Wasserstoff, Alkyl mit 1, 2 oder 3 C-Atomen oder Cycloalkyl mit 3 C-Atomen;
- R(2): Methoxy oder Ethoxy;
- R(3) und R(4): gleich oder verschieden Wasserstoff, Alkyl mit 5, 6, 7 oder 8 C-Atomen, Cycloalkyl mit 5 oder 6 C-Atomen oder eine (C₅₋₈H₁₁₋₁₇)-Gruppe, worin 1, 2, 3 oder 4 der CH₂-Gruppen durch Heteroatome O, S, NH ersetzt sind;
oder
- R(3) und R(4): gemeinsam (CH₂)₅₋₈, worin eine der CH₂-Gruppen durch Heteroatome O, S, NH ersetzt sein kann;
- E: Sauerstoff oder Schwefel;
- x: Sauerstoff;
- Y: [CR(5)R(5)')]ₘ;
R(5) und R(5')
unabhängig voneinander Wasserstoff oder Methyl, wobei die Glieder [CR(5)R(5')] gleich oder verschieden sind;
m 1 oder 2;
sowie deren pharmazeutisch verträgliche Salze.

Des weiteren ganz besonders bevorzugt sind die Verbindungen I, worin bedeuten:
- R(1): Wasserstoff, Alkyl mit 1, 2 oder 3 C-Atomen oder Cycloalkyl mit 3 C-Atomen;
- R(2): Methoxy oder Ethoxy;
- R(3): Wasserstoff;
- R(4): Alkyl mit 5, 6, 7 oder 8 C-Atomen, Cycloalkyl mit 5 oder 6 C-Atomen oder eine (C₅₋₈H₁₁₋₁₇)-Gruppe, in der 1, 2, 3 oder 4 CH₂-Gruppen durch Heteroatome 0, S, NH ersetzt sind;
- E: Schwefel oder Sauerstoff;
- x: Sauerstoff;
- Y: [CR(5)R(5')]ₘ;
R(5) und R(5')
unabhängig voneinander Wasserstoff oder Methyl;
wobei die Glieder [CR(5)R(5')] gleich oder verschieden sind;
m 1 oder 2;
sowie deren pharmazeutisch verträgliche Salze.

Die Verbindungen I der vorliegenden Erfindung sind wertvolle Arzneimittel zur Behandlung von Herzrhythmusstörungen unterschiedlichster Genese und zur Verhinderung des arrhythmisch bedingten, plötzlichen Herztods und können daher als Antiarrhythmika Verwendung finden. Beispiele arrhythmischer Störungen des Herzens sind supraventrikuläre Rhythmusstörungen, wie etwa Vorhof-Tachykardien, Vorhof-Flattern oder paroxysmale supraventrikuläre Rhythmusstörungen, oder ventrikuläre Rhythmusstörungen, wie ventrikuläre Extrasystolen, insbesondere aber lebensbedrohende ventrikuläre Tachykardien oder das besonders gefährliche Kammerflimmern. Sie eignen sich insbesondere für solche Fälle, in denen Arrhythmien Folge einer Verengung eines Koronargefäßes sind, wie sie beispielsweise bei Angina Pectoris oder während eines akuten Herzinfarkts oder als chronische Folge eines Herzinfarkts auftreten. Sie sind daher insbesondere bei Postinfarktpatienten zur Verhinderung des plötzlichen Herztods geeignet. Weitere Krankheitsbilder, wo derartige Rhythmusstörungen und/oder der plötzliche, arrhythmisch bedingte Herztod eine Rolle spielen sind beispielsweise die Herzinsuffizienz oder die Herzhypertrophie als Folge eines chronisch erhöhten Blutdrucks.

Darüber hinaus können die Verbindungen I eine verminderte Kontraktilität des Herzens positiv beeinflussen. Hierbei kann es sich um ein krankheitsbedingtes Nachlassen der Herzkontraktilität handeln, beispielsweise bei Herzinsuffizienz aber auch um akute Fälle wie Herzversagen bei Schockeinwirkungen. Ebenso kann bei einer Herztransplantation das Herz nach erfolgter Operation seine Leistungsfähigkeit rascher und zuverlässiger wieder aufnehmen. Gleiches gilt für Operationen am Herz, die eine vorübergehende Stillegung der Herzaktivität durch kardioplegische Lösungen erforderlich machen, wobei die Verbindungen sowohl für den Schutz der Organe beispielsweise bei Behandlung mit oder deren Lagerung in physiologischen Badflüssigkeiten, wie auch bei der Überführung in den Empfängerorganismus verwendet werden können.

Die Erfindung betrifft weiterhin Verfahren zur Herstellung der Verbindungen I. Man kann so vorgehen, daß man
(a) ein Sulfonamid der Formel II 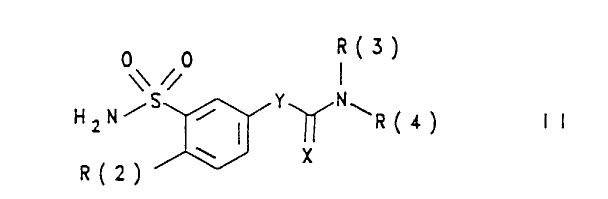 oder dessen Salz der Formel III 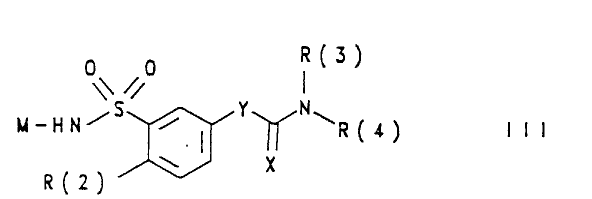 mit einem R(1)-substituierten Isocyanat der Formel IV

   R(1) - N = C = O IV

   worin R(1), R(2), R(3), R(4), X und Y die oben angegebenen Bedeutungen haben, zum substituierten Benzolsulfonylharnstoff I a umsetzt. Als Kationen M in den Salzen der Formel III kommen Alkali- und Erdalkaliionen in Betracht. Äquivalent zu den R(1)-substituierten Isocyanaten IV kann man R(1)-substituierte Carbamidsäureester, R(1)-substituierte Carbamidsäurehalogenide oder R(1)-substituierte Harnstoffe einsetzen;
(b) Ein Benzolsulfonylharnstoff der Formel I a 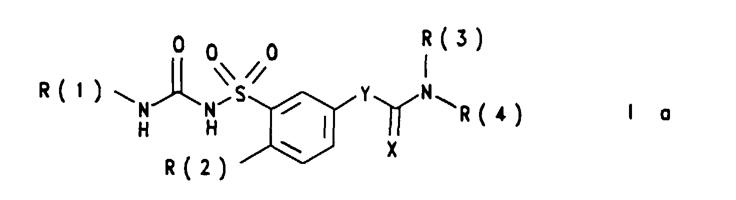 läßt sich aus einem aromatischen Benzolsulfonamid II oder dessen Salz III mit einem R(1)-substituierten Trichloracetamid der Formel V

   Cl₃C-C(=O)-NH-R(1) V

   in Gegenwart einer Base in einem inerten Lösungsmittel nach Synthesis 1987, 734 - 735 bei Temperaturen von 25°C bis 150°C herstellen.
   Als Basen eignen sich zum Beispiel Alkalimetall- oder Erdalkalimetallhydroxide, oder auch -alkoholate, wie Natriumhydroxid, Kaliumhydroxid, Calciumhydroxid, Natriummethylat, Natriumethanolat, Kaliummethylat oder Kaliumethanolat. Als inerte Lösungsmittel eignen sich Ether wie Tetrahydrofuran, Dioxan, Ethylenglykoldimethylether (Diglyme), Nitrile wie Acetonitril, Amide wie Dimethylformamid (DMF) oder N-Methylpyrrolidon (NMP), Phosphorsäurehexamethyltriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander.
(c) ein Benzolsulfonylthioharnstoff I b 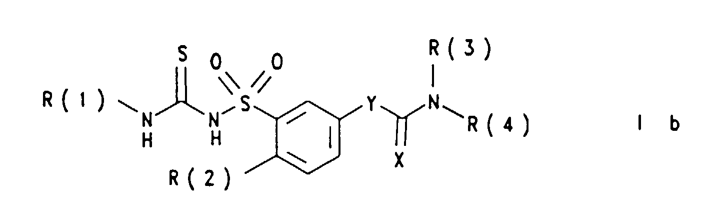 wird aus einem Benzolsulfonamid II sowie dessen Salze III und einem R(1)-substituierten Thioisocyanat VI

   R(1) - N = C = S VI

   hergestellt;
(d) Ein Benzolsulfonylharnstoff der Formel I a kann durch eine Umwandlungsreaktion von einem Benzolsulfonylthioharnstoff der Formel I b dargestellt werden. Der Ersatz des Schwefelatoms durch ein Sauerstoffatom im entsprechend substituierten Benzolsulfonylthioharnstoff I b kann beispielsweise mit Hilfe von Oxiden oder Salzen von Schwermetallen oder auch durch Anwendung von Oxidationsmitteln wie Wasserstoffperoxid, Natriumperoxid oder Salpetersäure ausgeführt werden. Thioharnstoffe können auch durch Behandlung mit Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Benzolsulfonylharnstoffe I a überführt werden. Isothioharnstoffe verhalten sich bei der Entschwefelung wie Thioharnstoffe und können demzufolge ebenso als Ausgangsstoffe für diese Reaktionen dienen.
(e) Ein Benzolsulfonylharnstoff I a läßt sich durch Umsetzung eines Amins der Formel R(1)-NH₂ mit einem Benzolsulfonylisocyanat der Formel VII 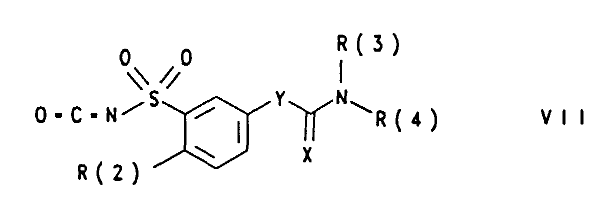 herstellen. Ebenso kann ein Amin R(1)-NH₂ mit einem Benzolsulfonylcarbamidsäureester, -carbamidsäurehalogenid oder Benzolsulfonylharnstoff I a [mit R(1) = H] zu einer Verbindung I a umgesetzt werden.
(f) Ein Benzolsulfonylthioharnstoff I b läßt sich durch Umsetzung eines Amins der Formel R(1)-NH₂ mit einem Benzolsulfonylisothiocyanat der Formel VIII 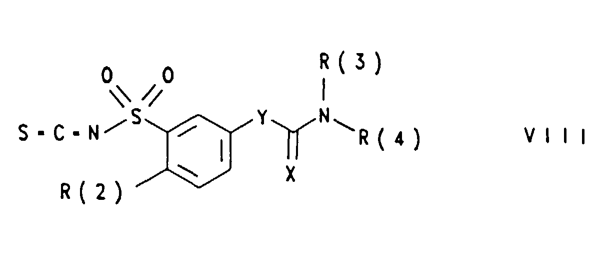 herstellen.
(g) Einen Benzolsulfonylharnstoff I a kann man aus einem Benzolsulfonylharnstoff der Formel IX 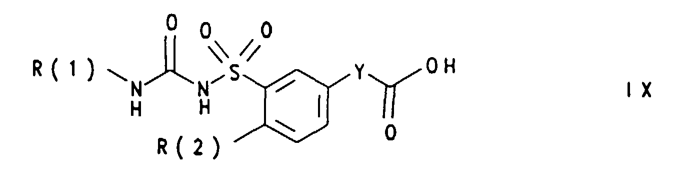 und R(3)R(4)NH mittels wasserentziehender Mittel oder Aktivierung mittels Carbonsäurehalogenide bzw. Bildung gemischter Anhydride darstellen.
   Als wasserentziehende Mittel können alle zur Herstellung von Amidbindungen geeignete Verbindungen eingesetzt werden, wie z.B. Dicyclohexylcarbodiimid, Carbonylbisimidazol, Propanphosphosphorsäureanhydrid. Als Lösungsmittel finden inerte nicht protische Lösungsmittel wie THF, DMF, Diethylether, Dichlormethan, sowie Gemische dieser Lösungsmittel Anwendung.
(h) Ein Benzolsulfonylthioharnstoff I b kann aus einem Benzolsulfonylthioharnstoff der Formel X 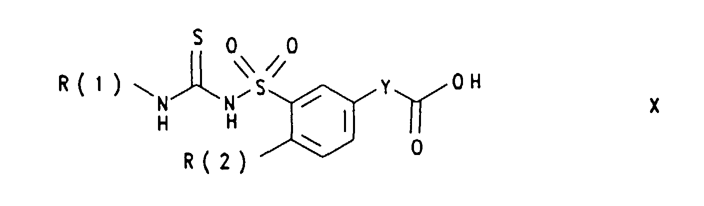 und R(3)R(4)NH mittels wasserentziehender Mittel oder Aktivierung mittels Carbonsäurehalogenide bzw. Bildung gemischter Anhydride dargestellt werden.
   Als wasserentziehende Mittel können alle zur Herstellung von Amidbindungen geeignete Verbindungen eingesetzt werden, wie z.B. Dicyclohexylcarbodiimid, Carbonylbisimidazol, Propanphosphosphorsäureanhydrid. Als Lösungsmittel finden inerte nicht protische Lösungsmittel wie THF, DMF, Diethylether, Dichlormethan, sowie Gemische dieser Lösungsmittel Anwendung.

Die Verbindungen I sowie deren physiologisch unbedenklichen Salze wertvolle Therapeutika, die sich nicht nur als Antiarrhythmika, sondern als Prophylaxe bei Störungen des kardiovaskulären Systems, Herzinsuffizienz, Herztranplantation oder cerebralen Gefäßerkrankungen an Menschen oder Säugetieren (zum Beispiel Affen, Hunde, Mäuse, Ratten, Kaninchen, Meerschweinchen und Katzen) eignen.

Unter physiologisch unbedenklichen Salzen der Verbindungen I versteht man nach Remmington's Pharmaceutical Science, 17. Auflage, 1985, Seiten 14-18 Verbindungen der Formel XI, 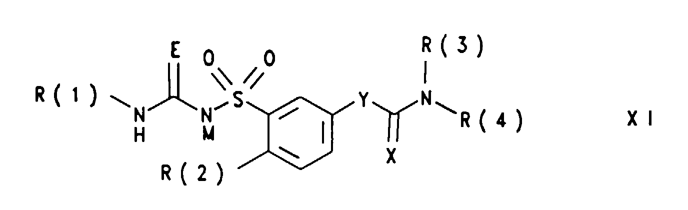 die sich aus nicht toxischen organischen und anorganischen Basen und Benzolsulfonylharnstoffen I darstellen lassen.

Bevorzugt werden hierbei Salze in denen M in der Formel XI Natrium, Kalium-, Rubidium-, Calcium-, Magnesiumionen sind, sowie die Säureadditionsprodukte basischer Aminosäuren, wie zum Beispiel Lysin oder Arginin.

Die Ausgangsverbindungen für die erwähnten Syntheseverfahren der Benzolsulfonylharnstoffe I werden nach bekannten Methoden hergestellt, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

So kann man eine geeignet substituierte Carbonsäure der Formel XII nach Schema 1 einer Halogensulfonierung unterwerfen und das durch anschließende Ammonolyse erhaltene Sulfonamid XIII mit einem entsprechenden Amin R(3)R(4)NH nach Aktivierung der Carbonsäuregruppe zum Carbonsäureamid der Formel II umsetzen.

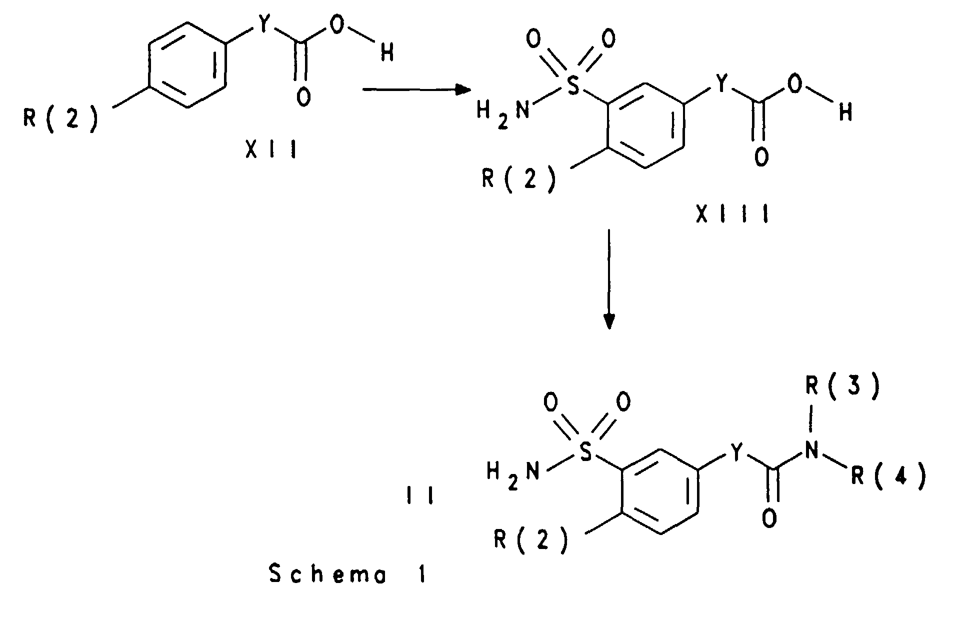

Als Aktivierungsmethoden eignen sich die Herstellung des Carbonsäurechlorids oder gemischter Carbonsäureanhydride mit Ameisensäurehalogeniden.
Desweiteren können die zur Amidbindungsherstellung bekannten Reagenzien wie zum Beispiel Carbonylbisimidazol, Dicyclohexylcarbodiimid und Propanphosphorsäureanhydrid angewandt werden.

Die in Schema 1 als Zwischenprodukte erhaltenen Sulfonamide XIII 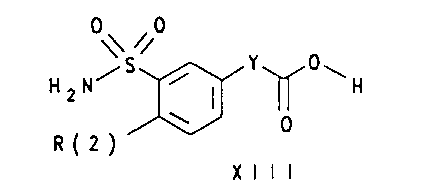 können mit entsprechenden Isocyanaten der Formel

R(1)-N=C=X

zu den Benzolsulfonylharnstoffcarbonsäuren der Formel XIV 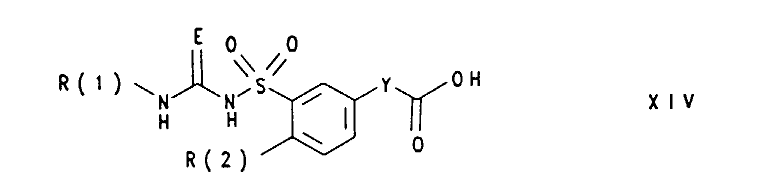 umgesetzt werden.

Die Verbindungen I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Isomere, beispielsweise durch Umkristallisieren aus inerten Lösungsmitteln, in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,R- und S- bzw. S,S-Formen von Weinsäure, Dibenzoylweinsäure, Diacetylweinsäure, Camphersulfonsäuren, Mandelsäuren, Äpfelsäure oder Milchsäure. Carbinole können ferner mit Hilfe chiraler Acylierungsreagenzien, zum Beispiel R- oder S-α-Methylbenzylisocyanat amidiert und dann getrennt werden. Die verschiedenen Formen der Diastereomeren können in bekannter Weise, zum Beispiel durch fraktionierte Kristallisation, getrennt und die Enantiomeren der Formel I können in bekannter Weise aus den Diastereomeren in Freiheit gesetzt werden.
Enantiomerentrennung gelingen ferner durch Chromatographie an optischaktiven Trägermaterialien.

Die erfindungsgemäßen Verbindungen I und ihre physiologisch unbedenklichen Salze können zur Herstellung pharmazeutischer Zubereitungen verwendet werden. Hierbei können sie zusammen mit mindestens einem festen, flüssigen Träger oder Hilfsstoff allein oder in Kombination mit anderen Herz-Kreislaufaktiven Arzneimitteln, wie etwa Calcium-Antagonisten, NO-Donatoren oder ACE-Hemmern in eine geeignete Dosierungsform gebracht werden. Diese Zubereitungen können als Arzneimittel in der Human- oder Veterinärmedizin verwendet werden. Als Trägerstoffe kommen organische oder anorganische Substanzen in Frage, die sich für die enterale (zum Beispiel orale), parenterale, wie zum Beispiel die intravenöse Applikation, oder topische Anwendungen eignen und mit den neuen Verbindungen nicht reagieren, beispielsweise Wasser, pflanzliche Öle, Benzylalkohole, Polyethylenglykole, Glycerintriacetat, Gelatine, Kohlenhydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Lösungen, vorzugsweise ölige oder wäßrige Lösungen, ferner Suspensionen, Emulsionen oder Implantate, für die topische Anwendung Salben, Cremes, Pasten, Lotionen, Gele, Sprays, Schäume, Aerosole, Lösungen (zum Beispiel in Alkoholen, wie Ethanol oder Isopropanol, Acetonitril, 1,2-Propandiol oder deren Gemische untereinander oder mit Wasser) oder Puder. Die Verbindungen I können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht, die Stabilisierungs- und/oder Netzmittel, Emulgatoren, Salze und/oder Hilfsstoffe wie Gleit-, Konservierungsmittel, Salze zur Beeinflussung des osmotischen Druckes, Puffersubstanzen, Farb- und Geschmacks- und/oder Aromastoffe enthalten. Sie können, falls erwünscht, auch einen oder mehrere weitere Wirkstoffe enthalten, zum Beispiel ein oder mehrere Vitamine.

Die Dosierungen, die zur Behandlung von Herzrhythmusstörungen mit den Verbindungen I notwendig sind, hängen davon ab, ob akut oder prophylaktisch therapiert wird. Normalerweise kommt man mit einem Dosisbereich von etwa mindestens 0.1 mg, vorzugsweise mindestens 1 mg, bis höchstens 100 mg, vorzugsweise höchstens 10 mg pro kg und Tag aus, wenn Prophylaxe betrieben wird. Bevorzugt ist ein Dosisbereich von 1 bis 10 mg pro kg und Tag. Die Dosis kann dabei als orale oder parenterale Einzeldosis oder in bis zu vier Einzeldosen aufgeteilt werden. Werden akute Fälle von Herzrhythmusstörungen behandelt, beispielsweise auf einer Intensivstation, kann die parenterale Verabreichung vorteilhaft sein. Ein bevorzugte Dosisbereich in kritischen Situationen kann dann vorzugsweise 10 bis 100 mg betragen und beispielsweise als intravenöse Dauerinfusion verabreicht werden.

Erfindungsgemäß können außer den in den Ausführungsbeispielen beschriebenen Verbindungen auch die in der folgenden Tabelle zusammengestellten Verbindungen I erhalten werden:
(1) 3-Sulfonylamino-N-(methylaminocarbonyl)-4-methoxyphenyl-essigsäure-N-methylamid,
(2) 3-Sulfonylamino-N-(methylaminocarbonyl)-4-methoxyphenyl-essigsäure-N-ethylamid
(3) 3-Sulfonylamino-N-(methylaminocarbonyl)-4-methoxyphenyl-essigsäure-N-1-propylamid
(4) 3-Sulfonylamino-N-(methylaminocarbonyl)-4-methoxyphenyl-essigsäure-N-2-propylamid
(5) 3-Sulfonylamino-N-(methylaminocarbonyl)-4-methoxyphenyl-essigsäure-N-1-butylamid
(6) 3-Sulfonylamino-N-(methylaminocarbonyl)-4-methoxyphenyl-essigsäure-N-2-butylamid
(7) 3-Sulfonylamino-N-(methylaminocarbonyl)-4-methoxyphenyl-essigsäure-N-1-pentylamid
(8) 3-Sulfonylamino-N-(methylaminocarbonyl)-4-methoxyphenyl-essigsäure-N-2-pentylamid
(9) 3-Sulfonylamino-N-(methylaminocarbonyl)-4-methoxyphenyl-essigsäure-N-3-pentylamid
(10) 3-Sulfonylamino-N-(methylaminocarbonyl)-4-methoxyphenyl-essigsäure-N-1-butyl-2-methylamid
(11) 3-Sulfonylamino-N-(methylaminocarbonyl)-4-methoxyphenyl-essigsäure-N-1-butyl-3-methylamid
(12) 3-Sulfonylamino-N-(methylaminocarbonyl)-4-methoxyphenyl-essigsäure-N-1-hexylamid
(13) 3-Sulfonylamino-N-(methylaminocarbonyl)-4-methoxyphenyl-essigsäure-N-2-hexylamid
(14) 3-Sulfonylamino-N-(methylaminocarbonyl)-4-methoxyphenyl-essigsäure-N-3-hexylamid
(15) 3-Sulfonylamino-N-(methylaminocarbonyl)-4-methoxyphenyl-essigsäure-N-1-heptylamid
(16) 3-Sulfonylamino-N-(methylaminocarbonyl)-4-methoxyphenyl-essigsäure-N-2-heptylamid
(17) 3-Sulfonylamino-N-(methylaminocarbonyl)-4-methoxyphenyl-essigsäure-N-3-heptylamid
(18) 3-Sulfonylamino-N-(methylaminocarbonyl)-4-methoxyphenyl-essigsäure-N-1-octylamid
(19) 3-Sulfonylamino-N-(methylaminocarbonyl)-4-methoxyphenyl-essigsäure-N-2-octylamid
(20) 3-Sulfonylamino-N-(methylaminocarbonyl)-4-methoxyphenyl-essigsäure-N-1-adamantylamid
(21) 3-Sulfonylamino-N-(methylaminocarbonyl)-4-methoxyphenyl-essigsäure-N-2-adamantylamid
(22) 3-Sulfonylamino-N-(methylaminocarbonyl)-4-methoxyphenyl-essigsäure-N-dimethylamid
(23) 3-Sulfonylamino-N-(methylaminocarbonyl)-4-methoxyphenyl-essigsäure-N-methyl-N'-ethylamid
(24) 3-Sulfonylamino-N-(methylaminocarbonyl)-4-methoxyphenyl-essigsäure-N-methyl-N'-1-propylamid
(25) 3-Sulfonylamino-N-(methylaminocarbonyl)-4-methoxyphenyl-essigsäure-N-methyl-N'-2-propylamid
(26) 3-Sulfonylamino-N-(methylaminocarbonyl)-4-methoxyphenyl-essigsäure-N-methyl-N'-1-butylamid
(27) 3-Sulfonylamino-N-(methylaminocarbonyl)-4-methoxyphenyl-essigsäure-N-methyl-N'-2-butylamid
(28) 3-Sulfonylamino-N-(methylaminocarbonyl)-4-methoxyphenyl-essigsäure-N-di-ethylamid
(29) 3-Sulfonylamino-N-(methylaminocarbonyl)-4-methoxyphenyl-essigsäure-N-ethyl-N'-1-propylamid
(30) 3-Sulfonylamino-N-(methylaminocarbonyl)-4-methoxyphenyl-essigsäure-N-ethyl-N'-2-propylamid
(31) 3-Sulfonylamino-N-(methylaminocarbonyl)-4-methoxyphenyl-essigsäurepyrrolidinylamid
(32) 3-Sulfonylamino-N-(methylaminocarbonyl)-4-methoxyphenyl-essigsäurepiperidylamid
(33) 3-Sulfonylamino-N-(methylaminocarbonyl)-4-methoxyphenyl-essigsäuremorpholinoamid
(34) 3-Sulfonylamino-N-(methylaminocarbonyl)-4-methoxyphenyl-essigsäure-(N-methyl-piperazinyl)amid
(35) 3-Sulfonylamino-N-(methylaminocarbonyl)-4-methoxyphenyl-essigsäure-4-thio-morpholinylamid
(36) 3-Sulfonylamino-N-(methylaminothiocarbonyl)-4-methoxyphenyl-essigsäure-N-methylamid,
(37) 3-Sulfonylamino-N-(methylaminothiocarbonyl)-4-methoxyphenyl-essigsäure-N-ethylamid
(38) 3-Sulfonylamino-N-(methylaminothiocarbonyl)-4-methoxyphenyl-essigsäure-N-1-propylamid
(39) 3-Sulfonylamino-N-(methylaminothiocarbonyl)-4-methoxyphenyl-essigsäure-N-2-propylamid
(40) 3-Sulfonylamino-N-(methylaminothiocarbonyl)-4-methoxyphenyl-essigsäure-N-1-butylamid
(41) 3-Sulfonylamino-N-(methylaminothiocarbonyl)-4-methoxyphenyl-essigsäure-N-2-butylamid
(42) 3-Sulfonylamino-N-(methylaminothiocarbonyl)-4-methoxyphenyl-essigsäure-N-1-pentylamid
(43) 3-Sulfonylamino-N-(methylaminothiocarbonyl)-4-methoxyphenyl-essigsäure-N-2-pentylamid
(44) 3-Sulfonylamino-N-(methylaminothiocarbonyl)-4-methoxyphenyl-essigsäure-N-3-pentylamid
(45) 3-Sulfonylamino-N-(methylaminothiocarbonyl)-4-methoxyphenyl-essigsäure-N-1-butyl-2-methylamid
(46) 3-Sulfonylamino-N-(methylaminothiocarbonyl)-4-methoxyphenyl-essigsäure-N-1-butyl-3-methylamid
(47) 3-Sulfonylamino-N-(methylaminothiocarbonyl)-4-methoxyphenyl-essigsäure-N-1-hexylamid
(48) 3-Sulfonylamino-N-(methylaminothiocarbonyl)-4-methoxyphenyl-essigsäure-N-2-hexylamid
(49) 3-Sulfonylamino-N-(methylaminothiocarbonyl)-4-methoxyphenyl-essigsäure-N-3-hexylamid
(50) 3-Sulfonylamino-N-(methylaminothiocarbonyl)-4-methoxyphenyl-essigsäure-N-1-heptylamid
(51) 3-Sulfonylamino-N-(methylaminothiocarbonyl)-4-methoxyphenyl-essigsäure-N-2-heptylamid
(52) 3-Sulfonylamino-N-(methylaminothiocarbonyl)-4-methoxyphenyl-essigsäure-N-3-heptylamid
(53) 3-Sulfonylamino-N-(methylaminothiocarbonyl)-4-methoxyphenyl-essigsäure-N-1-octylamid
(54) 3-Sulfonylamino-N-(methylaminothiocarbonyl)-4-methoxyphenyl-essigsäure-N-2-octylamid
(55) 3-Sulfonylamino-N-(methylaminothiocarbonyl)-4-methoxyphenyl-essigsäure-N-1-adamantylamid
(56) 3-Sulfonylamino-N-(methylaminothiocarbonyl)-4-methoxyphenyl-essigsäure-N-2-adamantylamid
(57) 3-Sulfonylamino-N-(methylaminothiocarbonyl)-4-methoxyphenyl-essigsäure-N-di-methyl-amid
(58) 3-Sulfonylamino-N-(methylaminothiocarbonyl)-4-methoxyphenyl-essigsäure-N-methyl-N'-ethylamid
(59) 3-Sulfonylamino-N-(methylaminothiocarbonyl)-4-methoxyphenyl-essigsäure-N-methyl-N'-1-propylamid
(60) 3-Sulfonylamino-N-(methylaminothiocarbonyl)-4-methoxyphenyl-essigsäure-N-methyl-N'-2-propylamid
(61) 3-Sulfonylamino-N-(methylaminothiocarbonyl)-4-methoxyphenyl-essigsäure-N-methyl-N'-1-butylamid
(62) 3-Sulfonylamino-N-(methylaminothiocarbonyl)-4-methoxyphenyl-essigsäure-N-methyl-N'-2-butylamid
(63) 3-Sulfonylamino-N-(methylaminothiocarbonyl)-4-methoxyphenyl-essigsäure-N-di-ethylamid
(64) 3-Sulfonylamino-N-(methylaminothiocarbonyl)-4-methoxyphenyl-essigsäure-N-ethyl-N'-1-propylamid
(65) 3-Sulfonylamino-N-(methylaminothiocarbonyl)-4-methoxyphenyl-essigsäure-N-ethyl-N'-2-propylamid
(66) 3-Sulfonylamino-N-(methylaminothiocarbonyl)-4-methoxyphenyl-essigsäure-pyrrolidinylamid
(67) 3-Sulfonylamino-N-(methylaminothiocarbonyl)-4-methoxyphenyl-essigsäure-piperidylamid
(68) 3-Sulfonylamino-N-(methylaminothiocarbonyl)-4-methoxyphenyl-essigsäure-morpholinoamid
(69) 3-Sulfonylamino-N-(methylaminothiocarbonyl)-4-methoxyphenyl-essigsäure-(N-methyl-piperazinyl)amid
(70) 3-Sulfonylamino-N-(methylaminothiocarbonyl)-4-methoxyphenyl-essigsäure-4-thio-morpholinylamid
(71) 3-Sulfonylamino-N-(methylaminocarbonyl)-4-methoxyphenyl-propionsäure-N-methylamid,
(72) 3-Sulfonylamino-N-(methylaminocarbonyl)-4-methoxyphenyl-propionsäure-N-ethylamid
(73) 3-Sulfonylamino-N-(methylaminocarbonyl)-4-methoxyphenyl-propionsäure-N-1-propylamid
(74) 3-Sulfonylamino-N-(methylaminocarbonyl)-4-methoxyphenyl-propionsäure-N-2-propylamid
(75) 3-Sulfonylamino-N-(methylaminocarbonyl)-4-methoxyphenyl-propionsäure-N-1-butylamid
(76) 3-Sulfonylamino-N-(methylaminocarbonyl)-4-methoxyphenyl-propionsäure-N-2-butylamid
(77) 3-Sulfonylamino-N-(methylaminocarbonyl)-4-methoxyphenyl-propionsäure-N-1-pentylamid
(78) 3-Sulfonylamino-N-(methylaminocarbonyl)-4-methoxyphenyl-propionsäure-N-2-pentylamid
(79) 3-Sulfonylamino-N-(methylaminocarbonyl)-4-methoxyphenyl-propionsäure-N-3-pentylamid
(80) 3-Sulfonylamino-N-(methylaminocarbonyl)-4-methoxyphenyl-propionsäure-N-1-butyl-2-methylamid
(81) 3-Sulfonylamino-N-(methylaminocarbonyl)-4-methoxyphenyl-propionsäure-N-1-butyl-3-methylamid
(82) 3-Sulfonylamino-N-(methylaminocarbonyl)-4-methoxyphenyl-propionsäure-N-1-hexylamid
(83) 3-Sulfonylamino-N-(methylaminocarbonyl)-4-methoxyphenyl-propionsäure-N-2-hexylamid
(84) 3-Sulfonylamino-N-(methylaminocarbonyl)-4-methoxyphenyl-propionsäure-N-3-hexylamid
(85) 3-Sulfonylamino-N-(methylaminocarbonyl)-4-methoxyphenyl-propionsäure-N-1-heptylamid
(86) 3-Sulfonylamino-N-(methylaminocarbonyl)-4-methoxyphenyl-propionsäure-N-2-heptylamid
(87) 3-Sulfonylamino-N-(methylaminocarbonyl)-4-methoxyphenyl-propionsäure-N-3-heptylamid
(88) 3-Sulfonylamino-N-(methylaminocarbonyl)-4-methoxyphenyl-propionsäure-N-1-octylamid
(89) 3-Sulfonylamino-N-(methylaminocarbonyl)-4-methoxyphenyl-propionsäure-N-2-octylamid
(90) 3-Sulfonylamino-N-(methylaminocarbonyl)-4-methoxyphenyl-propionsäure-N-1-adamantylamid
(91) 3-Sulfonylamino-N-(methylaminocarbonyl)-4-methoxyphenyl-propionsäure-N-2-adamantylamid
(92) 3-Sulfonylamino-N-(methylaminocarbonyl)-4-methoxyphenyl-propionsäure-N-di-methyl-amid
(93) 3-Sulfonylamino-N-(methylaminocarbonyl)-4-methoxyphenyl-propionsäure-N-methyl-N'-ethylamid
(94) 3-Sulfonylamino-N-(methylaminocarbonyl)-4-methoxyphenyl-propionsäure-N-methyl-N'-1-propylamid
(95) 3-Sulfonylamino-N-(methylaminocarbonyl)-4-methoxyphenyl-propionsäure-N-methyl-N'-2-propylamid
(96) 3-Sulfonylamino-N-(methylaminocarbonyl)-4-methoxyphenyl-propionsäure-N-methyl-N'-1-butylamid
(97) 3-Sulfonylamino-N-(methylaminocarbonyl)-4-methoxyphenyl-propionsäure-N-methyl-N'-2-butylamid
(98) 3-Sulfonylamino-N-(methylaminocarbonyl)-4-methoxyphenyl-propionsäure-N-di-ethylamid
(99) 3-Sulfonylamino-N-(methylaminocarbonyl)-4-methoxyphenyl-propionsäure-N-ethyl-N'-1-propylamid
(100) 3-Sulfonylamino-N-(methylaminocarbonyl)-4-methoxyphenyl-propionsäure-N-ethyl-N'-2-propylamid
(101) 3-Sulfonylamino-N-(methylaminocarbonyl)-4-methoxyphenyl-propionsäure-pyrrolidinylamid
(102) 3-Sulfonylamino-N-(methylaminocarbonyl)-4-methoxyphenyl-propionsäure-piperidylamid
(103) 3-Sulfonylamino-N-(methylaminocarbonyl)-4-methoxyphenyl-propionsäure-morpholinoamid
(104) 3-Sulfonylamino-N-(methylaminocarbonyl)-4-methoxyphenyl-propionsäure-(N-methyl-piperazinyl)amid
(105) 3-Sulfonylamino-N-(methylaminocarbonyl)-4-methoxyphenyl-propionsäure-4-thio-morpholinylamid
(106) 3-Sulfonylamino-N-(methylaminothiocarbonyl)-4-methoxyphenyl-propionsäure-N-methylamid,
(107) 3-Sulfonylamino-N-(methylaminothiocarbonyl)-4-methoxyphenyl-propionsäure-N-ethylamid
(108) 3-Sulfonylamino-N-(methylaminothiocarbonyl)-4-methoxyphenyl-propionsäure-N-1-propylamid
(109) 3-Sulfonylamino-N-(methylaminothiocarbonyl)-4-methoxyphenyl-propionsäure-N-2-propylamid
(110) 3-Sulfonylamino-N-(methylaminothiocarbonyl)-4-methoxyphenyl-propionsäure-N-1-butylamid
(111) 3-Sulfonylamino-N-(methylaminothiocarbonyl)-4-methoxyphenyl-propionsäure-N-2-butylamid
(112) 3-Sulfonylamino-N-(methylaminothiocarbonyl)-4-methoxyphenyl-propionsäure-N-1-pentylamid
(113) 3-Sulfonylamino-N-(methylaminothiocarbonyl)-4-methoxyphenyl-propionsäure-N-2-pentylamid
(114) 3-Sulfonylamino-N-(methylaminothiocarbonyl)-4-methoxyphenyl-propionsäure-N-3-pentylamid
(115) 3-Sulfonylamino-N-(methylaminothiocarbonyl)-4-methoxyphenyl-propionsäure-N-1-butyl-2-methylamid
(116) 3-Sulfonylamino-N-(methylaminothiocarbonyl)-4-methoxyphenyl-propionsäure-N-1-butyl-3-methylamid
(117) 3-Sulfonylamino-N-(methylaminothiocarbonyl)-4-methoxyphenyl-propionsäure-N-1-hexylamid
(118) 3-Sulfonylamino-N-(methylaminothiocarbonyl)-4-methoxyphenyl-propionsäure-N-2-hexylamid
(119) 3-Sulfonylamino-N-(methylaminothiocarbonyl)-4-methoxyphenyl-propionsäure-N-3-hexylamid
(120) 3-Sulfonylamino-N-(methylaminothiocarbonyl)-4-methoxyphenyl-propionsäure-N-1 -heptylamid
(121) 3-Sulfonylamino-N-(methylaminothiocarbonyl)-4-methoxyphenyl-propionsäure-N-2-heptylamid
(122) 3-Sulfonylamino-N-(methylaminothiocarbonyl)-4-methoxyphenyl-propionsäure-N-3-heptylamid
(123) 3-Sulfonylamino-N-(methylaminothiocarbonyl)-4-methoxyphenyl-propionsäure-N-1-octylamid
(124) 3-Sulfonylamino-N-(methylaminothiocarbonyl)-4-methoxyphenyl-propionsäure-N-2-octylamid
(125) 3-Sulfonylamino-N-(methylaminothiocarbonyl)-4-methoxyphenyl-propionsäure-N-1-adamantylamid
(126) 3-Sulfonylamino-N-(methylaminothiocarbonyl)-4-methoxyphenyl-propionsäure-N-2-adamantylamid
(127) 3-Sulfonylamino-N-(methylaminothiocarbonyl)-4-methoxyphenyl-propionsäure-N-di-methyl-amid
(128) 3-Sulfonylamino-N-(methylaminothiocarbonyl)-4-methoxyphenyl-propionsäure-N-methyl-N'-ethylamid
(129) 3-Sulfonylamino-N-(methylaminothiocarbonyl)-4-methoxyphenylpropionsäure-N-methyl-N'-1-propylamid
(130) 3-Sulfonylamino-N-(methylaminothiocarbonyl)-4-methoxyphenylpropionsäure-N-methyl-N'-2-propylamid
(131) 3-Sulfonylamino-N-(methylaminothiocarbonyl)-4-methoxyphenylpropionsäure-N-methyl-N'-1-butylamid
(132) 3-Sulfonylamino-N-(methylaminothiocarbonyl)-4-methoxyphenylpropionsäure-N-methyl-N'-2-butylamid
(133) 3-Sulfonylamino-N-(methylaminothiocarbonyl)-4-methoxyphenylpropionsäure-N-di-ethylamid
(134) 3-Sulfonylamino-N-(methylaminothiocarbonyl)-4-methoxyphenylpropionsäure-N-ethyl-N'-1-propylamid
(135) 3-Sulfonylamino-N-(methylaminothiocarbonyl)-4-methoxyphenylpropionsäure-N-ethyl-N'-2-propylamid
(136) 3-Sulfonylamino-N-(methylaminothiocarbonyl)-4-methoxyphenylpropionsäure-pyrrolidinylamid
(137) 3-Sulfonylamino-N-(methylaminothiocarbonyl)-4-methoxyphenylpropionsäure-piperidylamid
(138) 3-Sulfonylamino-N-(methylaminothiocarbonyl)-4-methoxyphenylpropionsäure-morpholinoamid
(139) 3-Sulfonylamino-N-(methylaminothiocarbonyl)-4-methoxyphenylpropionsäure-(N-methyl-piperazinyl)amid
(140) 3-Sulfonylamino-N-(methylaminothiocarbonyl)-4-methoxyphenylpropionsäure-4-thio-morpholinylamid
(141) 3-Sulfonylamino-N-(methylaminothiocarbonyl)-4-methylphenyl-essigsäure-N-methylamid,
(142) 3-Sulfonylamino-N-(methylaminothiocarbonyl)-4-methylphenyl-essigsäure-N-ethylamid
(143) 3-Sulfonylamino-N-(methylaminothiocarbonyl)-4-methylphenyl-essigsäure-N-1-propylamid
(144) 3-Sulfonylamino-N-(methylaminothiocarbonyl)-4-methylphenyl-essigsäure-N-2-propylamid
(145) 3-Sulfonylamino-N-(methylaminothiocarbonyl)-4-methylphenyl-essigsäure-N-1-butylamid
(146) 3-Sulfonylamino-N-(methylaminothiocarbonyl)-4-methylphenyl-essigsäure-N-2-butylamid
(147) 3-Sulfonylamino-N-(methylaminothiocarbonyl)-4-methylphenyl-essigsäure-N-1-pentylamid
(148) 3-Sulfonylamino-N-(methylaminothiocarbonyl)-4-methylphenyl-essigsäure-N-2-pentylamid
(149) 3-Sulfonylamino-N-(methylaminothiocarbonyl)-4-methylphenyl-essigsäure-N-3-pentylamid
(150) 3-Sulfonylamino-N-(methylaminothiocarbonyl)-4-methylphenyl-essigsäure-N-1-butyl-2-methylamid
(151) 3-Sulfonylamino-N-(methylaminothiocarbonyl)-4-methylphenyl-essigsäure-N-1-butyl-3-methylamid
(152) 3-Sulfonylamino-N-(methylaminothiocarbonyl)-4-methylphenyl-essigsäure-N-1-hexylamid
(153) 3-Sulfonylamino-N-(methylaminothiocarbonyl)-4-methylphenyl-essigsäure-N-2-hexylamid
(154) 3-Sulfonylamino-N-(methylaminothiocarbonyl)-4-methylphenyl-essigsäure-N-3-hexylamid
(155) 3-Sulfonylamino-N-(methylaminothiocarbonyl)-4-methylphenyl-essigsäure-N-1-heptylamid
(156) 3-Sulfonylamino-N-(methylaminothiocarbonyl)-4-methylphenyl-essigsäure-N-2-heptylamid
(157) 3-Sulfonylamino-N-(methylaminothiocarbonyl)-4-methylphenyl-essigsäure-N-3-heptylamid
(158) 3-Sulfonylamino-N-(methylaminothiocarbonyl)-4-methylphenyl-essigsäure-N-1-octylamid
(159) 3-Sulfonylamino-N-(methylaminothiocarbonyl)-4-methylphenyl-essigsäure-N-2-octylamid
(160) 3-Sulfonylamino-N-(methylaminothiocarbonyl)-4-methylphenyl-essigsäure-N-1-adamantylamid
(161) 3-Sulfonylamino-N-(methylaminothiocarbonyl)-4-methylphenyl-essigsäure-N-2-adamantylamid
(162) 3-Sulfonylamino-N-(methylaminothiocarbonyl)-4-methylphenyl-essigsäure-N-di-methyl-amid
(163) 3-Sulfonylamino-N-(methylaminothiocarbonyl)-4-methylphenyl-essigsäure-N-methyl-N'-ethylamid
(164) 3-Sulfonylamino-N-(methylaminothiocarbonyl)-4-methylphenyl-essigsäure-N-methyl-N'-1-propylamid
(165) 3-Sulfonylamino-N-(methylaminothiocarbonyl)-4-methylphenyl-essigsäure-N-methyl-N'-2-propylamid
(166) 3-Sulfonylamino-N-(methylaminothiocarbonyl)-4-methylphenyl-essigsäure-N-methyl-N'-1-butylamid
(167) 3-Sulfonylamino-N-(methylaminothiocarbonyl)-4-methylphenyl-essigsäure-N-methyl-N'-2-butylamid
(168) 3-Sulfonylamino-N-(methylaminothiocarbonyl)-4-methylphenyl-essigsäure-N-di-ethylamid
(169) 3-Sulfonylamino-N-(methylaminothiocarbonyl)-4-methylphenyl-essigsäure-N-ethyl-N'-1-propylamid
(170) 3-Sulfonylamino-N-(methylaminothiocarbonyl)-4-methylphenyl-essigsäure-N-ethyl-N'-2-propylamid
(171) 3-Sulfonylamino-N-(methylaminothiocarbonyl)-4-methylphenyl-essigsäure-pyrrolidinylamid
(172) 3-Sulfonylamino-N-(methylaminothiocarbonyl)-4-methylphenyl-essigsäure-piperidylamid
(173) 3-Sulfonylamino-N-(methylaminothiocarbonyl)-4-methylphenyl-essigsäure-morpholinoamid
(174) 3-Sulfonylamino-N-(methylaminothiocarbonyl)-4-methylphenyl-essigsäure-(N-methyl-piperazinyl)amid
(175) 3-Sulfonylamino-N-(methylaminothiocarbonyl)-4-methylphenyl-essigsäure-4-thio-morpholinylamid
(176) 3-Sulfonylamino-N-(methylaminothiocarbonyl)-4-methylphenyl-propionsäure-N-methylamid,
(177) 3-Sulfonylamino-N-(methylaminothiocarbonyl)-4-methylphenylpropionsäure-N-ethylamid
(178) 3-Sulfonylamino-N-(methylaminothiocarbonyl)-4-methylphenylpropionsäure-N-1-propylamid
(179) 3-Sulfonylamino-N-(methylaminothiocarbonyl)-4-methylphenylpropionsäure-N-2-propylamid
(180) 3-Sulfonylamino-N-(methylaminothiocarbonyl)-4-methylphenylpropionsäure-N-1-butylamid
(181) 3-Sulfonylamino-N-(methylaminothiocarbonyl)-4-methylphenylpropionsäure-N-2-butylamid
(182) 3-Sulfonylamino-N-(methylaminothiocarbonyl)-4-methylphenylpropionsäure-N-1-pentylamid
(183) 3-Sulfonylamino-N-(methylaminothiocarbonyl)-4-methylphenylpropionsäure-N-2-pentylamid
(184) 3-Sulfonylamino-N-(methylaminothiocarbonyl)-4-methylphenylpropionsäure-N-3-pentylamid
(185) 3-Sulfonylamino-N-(methylaminothiocarbonyl)-4-methylphenylpropionsäure-N-1-butyl-2-methylamid
(186) 3-Sulfonylamino-N-(methylaminothiocarbonyl)-4-methylphenylpropionsäure-N-1-butyl-3-methylamid
(187) 3-Sulfonylamino-N-(methylaminothiocarbonyl)-4-methylphenylpropionsäure-N-1-hexylamid
(188) 3-Sulfonylamino-N-(methylaminothiocarbonyl)-4-methylphenylpropionsäure-N-2-hexylamid
(189) 3-Sulfonylamino-N-(methylaminothiocarbonyl)-4-methylphenylpropionsäure-N-3-hexylamid
(190) 3-Sulfonylamino-N-(methylaminothiocarbonyl)-4-methylphenylpropionsäure-N-1-heptylamid
(191) 3-Sulfonylamino-N-(methylaminothiocarbonyl)-4-methylphenylpropionsäure-N-2-heptylamid
(192) 3-Sulfonylamino-N-(methylaminothiocarbonyl)-4-methylphenylpropionsäure-N-3-heptylamid
(193) 3-Sulfonylamino-N-(methylaminothiocarbonyl)-4-methylphenylpropionsäure-N-1-octylamid
(194) 3-Sulfonylamino-N-(methylaminothiocarbonyl)-4-methylphenylpropionsäure-N-2-octylamid
(195) 3-Sulfonylamino-N-(methylaminothiocarbonyl)-4-methylphenylpropionsäure-N-1-adamantylamid
(196) 3-Sulfonylamino-N-(methylaminothiocarbonyl)-4-methylphenylpropionsäure-N-2-adamantylamid
(197) 3-Sulfonylamino-N-(methylaminothiocarbonyl)-4-methylphenylpropionsäure-N-di-methyl-amid
(198) 3-Sulfonylamino-N-(methylaminothiocarbonyl)-4-methylphenylpropionsäure-N-methyl-N'-ethylamid
(199) 3-Sulfonylamino-N-(methylaminothiocarbonyl)-4-methylphenylpropionsäure-N-methyl-N'-1-propylamid
(200) 3-Sulfonylamino-N-(methylaminothiocarbonyl)-4-methylphenylpropionsäure-N-methyl-N'-2-propylamid
(201) 3-Sulfonylamino-N-(methylaminothiocarbonyl)-4-methylphenylpropionsäure-N-methyl-N'-1-butylamid
(202) 3-Sulfonylamino-N-(methylaminothiocarbonyl)-4-methylphenylpropionsäure-N-methyl-N'-2-butylamid
(203) 3-Sulfonylamino-N-(methylaminothiocarbonyl)-4-methylphenylpropionsäure-N-di-ethylamid
(204) 3-Sulfonylamino-N-(methylaminothiocarbonyl)-4-methylphenylpropionsäure-N-ethyl-N'-1-propylamid
(205) 3-Sulfonylamino-N-(methylaminothiocarbonyl)-4-methylphenylpropionsäure-N-ethyl-N'-2-propylamid
(206) 3-Sulfonylamino-N-(methylaminothiocarbonyl)-4-methylphenylpropionsäure-pyrrolidinylamid
(207) 3-Sulfonylamino-N-(methylaminothiocarbonyl)-4-methylphenylpropionsäure-piperidylamid
(208) 3-Sulfonylamino-N-(methylaminothiocarbonyl)-4-methylphenylpropionsäure-morpholinoamid
(209) 3-Sulfonylamino-N-(methylaminothiocarbonyl)-4-methylphenylpropionsäure-(N-methyl-piperazinyl)amid
(210) 3-Sulfonylamino-N-(methylaminothiocarbonyl)-4-methylphenylpropionsäure-4-thio-morpholinylamid

### Herstellung der Ausgangsmaterialien

### Herstellung von 3-Sulfonylamino-phenyl-carbonsäuren

Die 4-substituierten Phenylcarbonsäuren werden unter Rühren portionsweise zu einem Überschuß an Chlorsulfonsäure gegeben. Man rührt 30 min bei Raumtemperatur, gießt anschließend auf Eis und saugt das entstandene Sulfonsäurechlorid ab. Dieses wird in Ammoniaklösung gelöst, 30 min bei Raumtemperatur gerührt und die Lösung mit 2 N Salzsäure neutralisiert. Das erhaltene Produkt wird abgesaugt. Nach dieser Methode hergestellt:

### 3-Sulfonylamino-4-methoxy-phenyl-3-propionsäure

Smp. 172 - 176°C

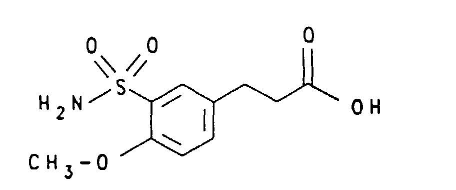

### 3-Sulfonylamino-4-methoxy-phenylessigsäure

Smp. 164°C

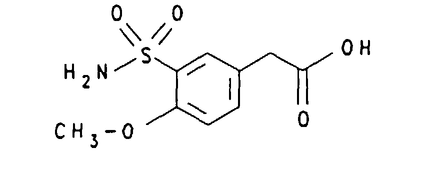

### 3-Sulfonylamino-4-ethoxy-phenylessigsäure

Smp. 183 - 185°C

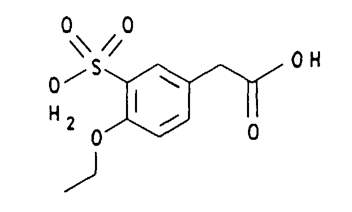

### Herstellung von 3-Sulfonylamino-N-(methylaminothiocarbonyl)-4-methoxyphenyl-essigsäure

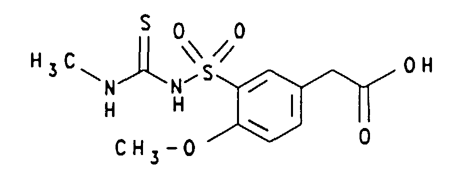

5 g 3-Sulfonylamino-4-methoxy-phenyl-essigsäure werden in 3 ml DMF gelöst und mit 245 mg Natriumhydroxid für 30 min bei 40°C gerührt. Hierzu gibt man 328 mg Methylisothiocyanat und rührt weitere 2 h bei 70°C. Zu der abgekühlten Lösung gibt man 2 N Salzsäure und saugt das Produkt ab. Smp. 174°C.

Nach analoger Vorschrift wurde hergestellt:

### 3-Sulfonylamino-N-(methylaminothiocarbonyl)-4-ethoxyphenyl-essigsäure

Smp. 152 - 154°C

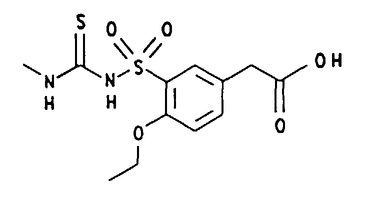

### Beispiele:

Beispiel 1: 3-Sulfonylamino-N-(methylaminothiocarbonyl)-4-methoxyphenylpropionsäure-cyclohexylamid 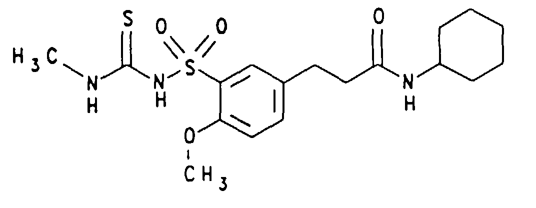 500 mg 3-Sulfonylamino-4-methoxyphenyl-propionsäure-cyclohexylamid werden in 10 ml DMF gelöst, mit 88 mg NaOH versetzt und 30 min bei 40°C gerührt. Anschließend gibt man 107 mg Methylisothiocyanat zu und rührt weitere 2 h bei 70°C. Nach Abkühlen und Neutralisieren mit 2 N Salzsäure saugt man das Produkt ab und trocknet.
Smp. 163°C.

Beispiel 2: 3-Sulfonylamino-N-(methylaminothiocarbonyl)-4-methoxyphenylessigsäure-cyclohexylamid 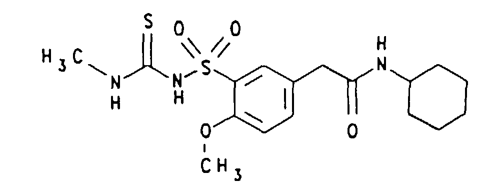 Analog werden aus 500 mg 3-Sulfonylamino-4-methoxyphenyl-essigsäurecyclohexylamid 360 mg 3-Sulfonylamino-N-(methylaminothiocarbonyl)-4-methoxyphenyl-essigsäure-cyclohexylamid hergestellt.
Smp. 185°C.

Beispiel 3: 3-Sulfonylamino-N-(methylaminothiocarbonyl)-4-methoxyphenylessigsäure-N-3-pentylamid 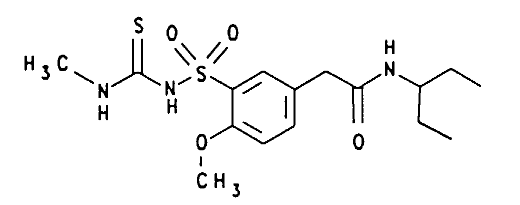 400 mg 3-Sulfonylamino-N-(methylaminothiocarbonyl)-4-methoxyphenylessigsäure werden in 5 ml THF gelöst und mit 224 mg Carbonylbisimidazol versetzt. Man rührt 2 h bei Raumtemperatur. Nach der Zugabe von 120 mg 3-Aminopentan wird über Nacht bei Raumtemperatur gerührt. Man reduziert die Lösungsmittelmenge im Vakuum und gibt den Rückstand auf 2 N HCI. Der Feststoff wird abgesaugt.
Smp. 169°C.

Beispiel 4: 3-Sulfonylamino-N-(methylaminothiocarbonyl)-4-methoxyphenylessigsäure-N-(R)-1-cyclohexyl-1-ethylamid 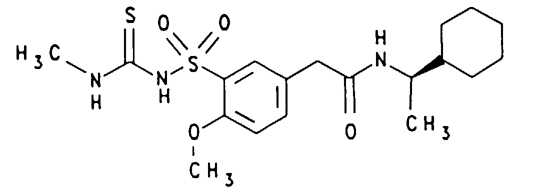 Analog Beispiel 3 wird aus 300 mg 3-Sulfonylamino-N-(methylaminothiocarbonyl)-4-methoxyphenyl-essigsäure und 144 mg (R)-1-Cyclohexyl-1-ethylamin das 3-Sulfonylamino-N-(methylaminothiocarbonyl)-4-methoxyphenyl-essigsäure-N-(R)-1-cyclohexyl-1-ethylamid gewonnen. Smp. 84°C.

Beispiel 5: 3-Sulfonylamino-N-(methylaminothiocarbonyl)-4-methoxyphenylessigsäure-N-(S)-1-cyclohexyl-1-ethylamid 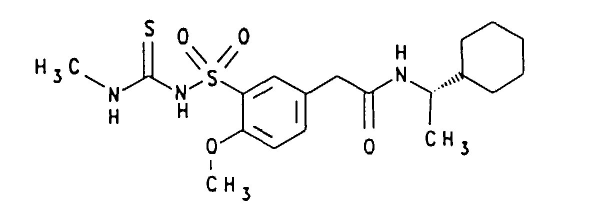 Analog Beispiel 3 wird aus 300 mg 3-Sulfonylamino-N-(methylaminothiocarbonyl)-4-methoxyphenyl-essigsäure und 144 mg (S)-1-Cyclohexyl-1-ethylamin das 3-Sulfonylamino-N-(methylaminothiocarbonyl)-4-methoxyphenyl-essigsäure-N-(S)-1-cyclohexyl-1-ethylamid gewonnen. Smp. 84°C.

Beispiel 6: 3-Sulfonylamino-N-(methylaminothiocarbonyl)-4-methoxyphenylessigsäure-N-1-butylamid

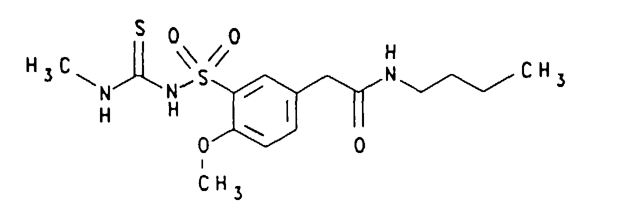

Analog Beispiel 3 wird das Produkt aus 300 mg 3-Sulfonylamino-N-(methylaminothiocarbonyl)-4-methoxyphenyl-essigsäure und 83 mg Butylamin gewonnen.
Smp. 136°C.

Beispiel 7: 3-Sulfonylamino-N-(methylaminothiocarbonyl)-4-methoxyphenyl-3-propionsäure-n-isopropylamid 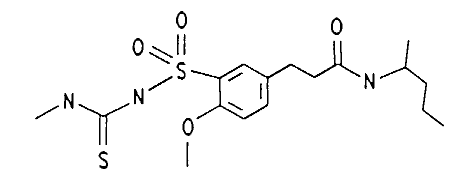 Smp: 116°C

Beispiel 8: 3-Sulfonylamino-N-(methylaminothiocarbonyl)-4-methoxyphenyl-3-propionsäure-n-isopropylamid 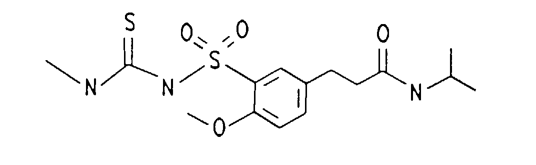 Smp: 172°C

Beispiel 9: 3-Sulfonylamino-N-(methylaminothiocarbonyl)-4-methoxyphenyl-3-essigsäure-n-2-pentylamid 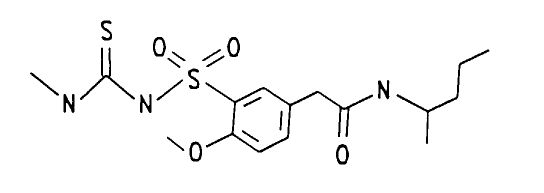 Smp: 137°C

Beispiel 10: 3-Sulfonylamino-N-lmethylaminothiocarbonyl)-4-methoxyphenyl-3-essigsäure-n-isopropylamid 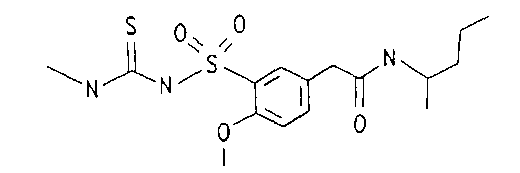 Öl.

Beispiel 11: 3-Sulfonylamino-N-(methylaminothiocarbonyl)-4-methoxyphenylessigsäure-n-2-(R)-butylamid 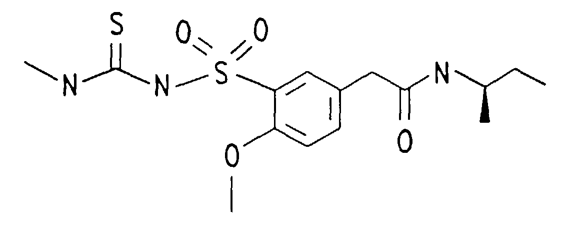 Smp: 123°C

Beispiel 12: 3-Sulfonylamino-N-(methylaminothiocarbonyl)-4-methoxyphenylessigsäure-n-2-(S)-butylamid 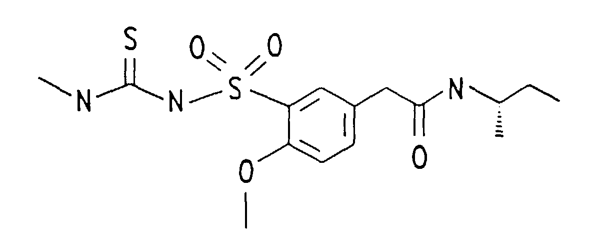 Smp: 119°C

Beispiel 13: 3-Sulfonylamino-N-(methylaminothiocarbonyl)-4-methoxyphenylessigsäure-n-4-heptylamid 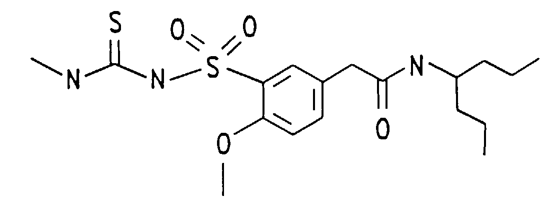 Smp: 118°C

Beispiel 14: 3-Sulfonylamino-N-(methylaminothiocarbonyl)-4-methoxyphenylessigsäure-n-6-methyl-2-heptylamid 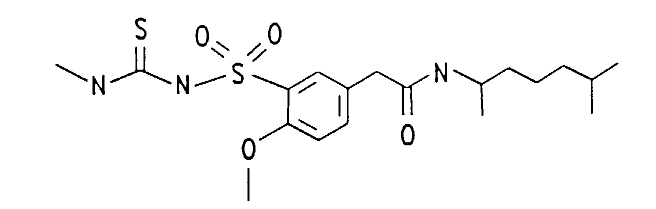 Smp: 113°C

Beispiel 15: 3-Sulfonylamino-N-(methylaminothiocarbonyl)-4-methoxyphenylessigsäure-n-2-undecylamid 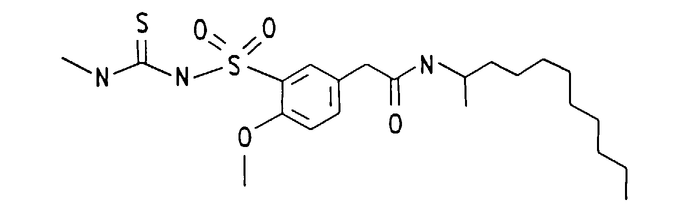 Beispiel 16: 3-Sulfonylamino-N-(methylaminothiocarbonyl)-4-methoxyphenylessigsäure-n-2-hexylamid 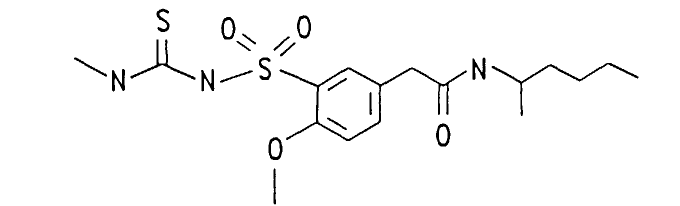 Smp: 111°C

Beispiel 17: 3-Sulfonylamino-N-(methylaminothiocarbonyl)-4-methoxyphenylessigsäure-n-3,3-dimethyl-2-butylamid 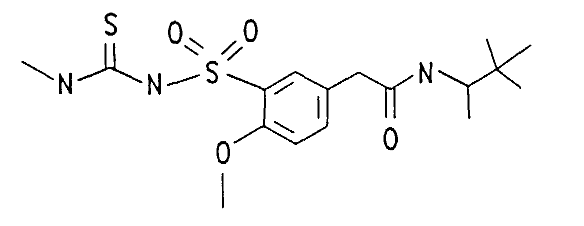 Smp: 170°C

Beispiel 18: 3-Sulfonylamino-N-(methylaminothiocarbonyl)-4-methoxyphenylessigsäure-n-2-(S)-heptylamid 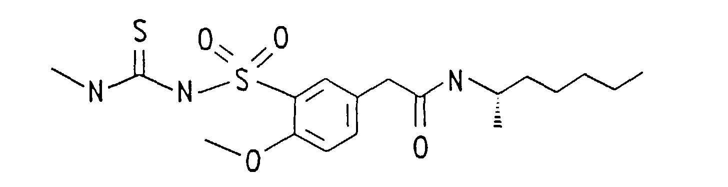 Smp: 139°C

Beispiel 19: 3-Sulfonylamino-N-(methylaminothiocarbonyl)-4-methoxyphenylessigsäure-n-2-(R)-heptylamid 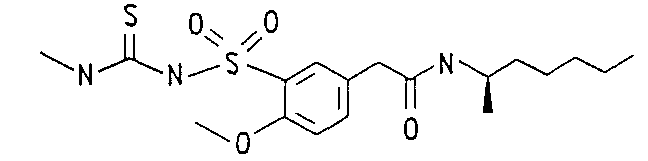 Smp: 138°C

Beispiel 20: 3-Sulfonylamino-N-(methylaminothiocarbonyl)-4-methoxyphenylessigsäure-n-2-octylamid 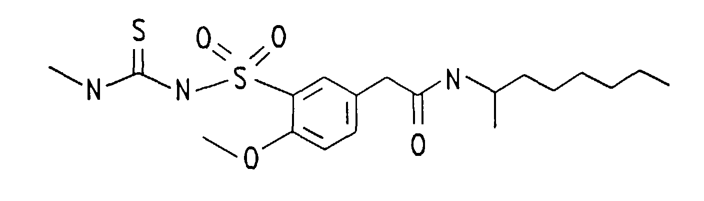 Smp: 103°C

Beispiel 21: 3-Sulfonylamino-N-(methylaminothiocarbonyl)-4-methoxyphenylessigsäure-n-6-(2-methyl)-heptan-2-ol-amid 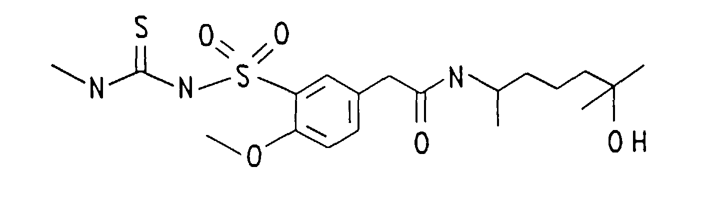 Smp: 85°C

Beispiel 22: 3-Sulfonylamino-N-(methylaminothiocarbonyl)-4-methoxyphenylessigsäure-n-2-(S)-pentylamid 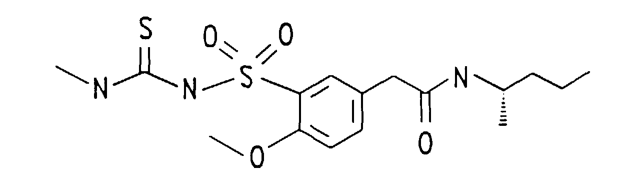 Smp: 102°C

Beispiel 23: 3-Sulfonylamino-N-(methylaminothiocarbonyl)-4-ethoxyphenylessigsäure-n-4-heptylamid 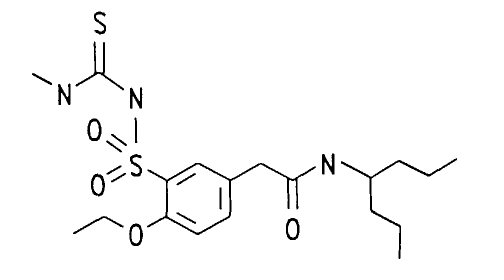 Smp: 157°C

Beispiel 24: 3-Sulfonylamino-N-(methylaminothiocarbonyl)-4-ethoxyphenylessigsäure-n-(S)-1-cyclohexylethylamid 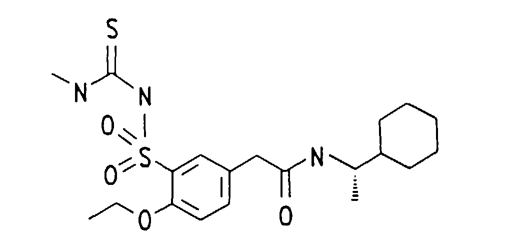 Smp: 162°C

Beispiel 25: 3-Sulfonylamino-N-(methylaminothiocarbonyl)-4-ethoxyphenylessigsäure-n-6-methyl-2-heptylamid 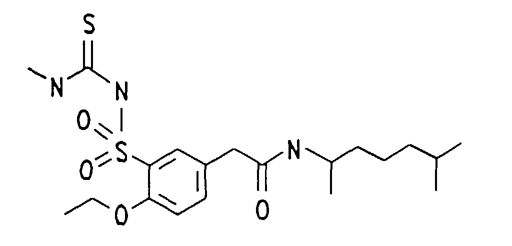 Smp: 122°C

Beispiel 26: 3-Sulfonylamino-N-(methylaminothiocarbonyl)-4-ethoxyphenylessigsäure-n-2-(S)-heptylamid 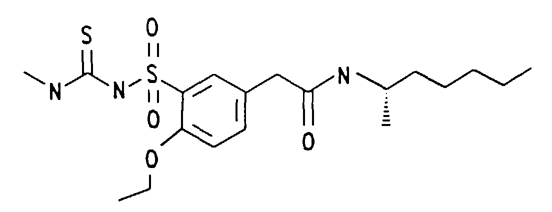 Smp: 137°C

Beispiel 27: 3-Sulfonylamino-N-(methylaminocarbonyl)-4-ethoxyphenylessigsäure-n-2-(S)-heptylamid 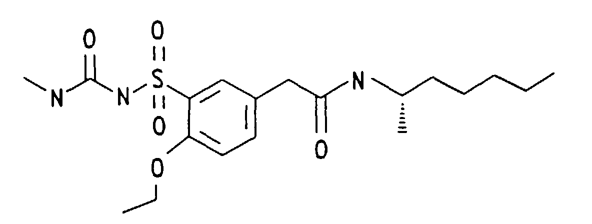 Smp: 151°C

Beispiel 28: 3-Sulfonylamino-N-(methylaminothiocarbonyl)-4-ethoxyphenyl-essigsäure-n-2-octylamid 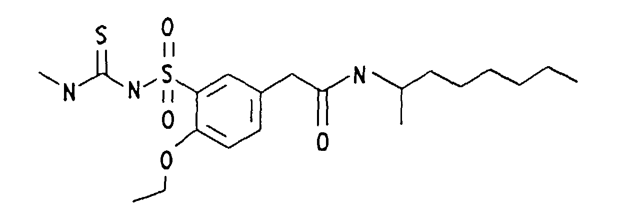 Smp: 123°C

Beispiel 29: 3-Sulfonylamino-N-(methylaminothiocarbonyl)-4-ethoxyphenylessigsäure-n-1-adamantylamid 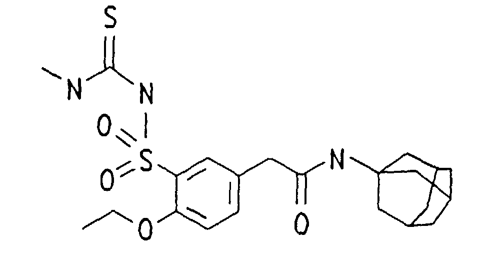 Smp: 170°C

Beispiel 30: 3-Sulfonylamino-N-(methylaminothiocarbonyl)-4-methoxyphenylessigsäure-n-n-heptylamid 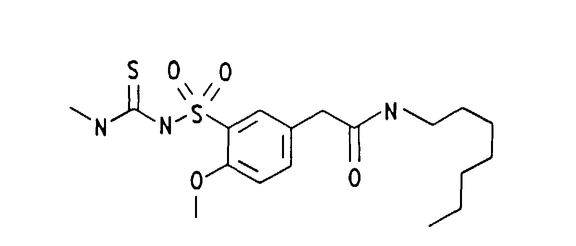 Smp: 139°C

Beispiel 31: 3-Sulfonylamino-N-(methylaminothiocarbonyl)-4-methoxyphenylessigsäure-n-n-octylamid 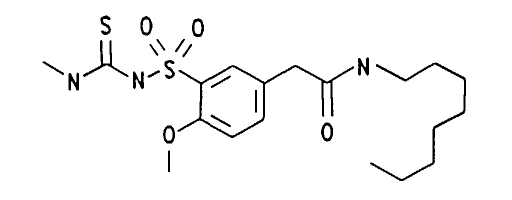 Smp: 132°C

Beispiel 32: 3-Sulfonylamino-N-(methylaminothiocarbonyl)-4-methoxyphenylessigsäure-n-n-nonylamid 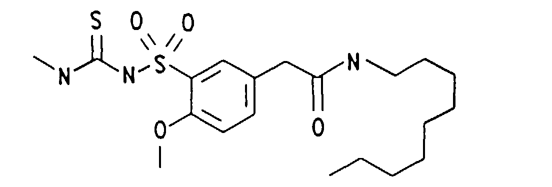 Smp: 134°C

Beispiel 33: 3-Sulfonylamino-N-(methylaminocarbonyl)-4-methoxyphenylessigsäure-n-2-(S)-heptylamid 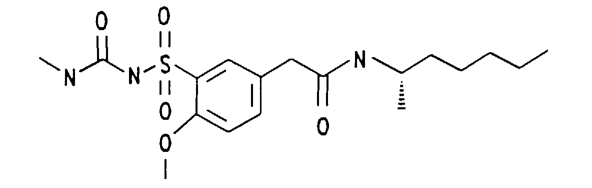 Smp: 178°C

Beispiel 34: 3-Sulfonylamino-N-(methylaminocarbonyl)-4-methoxyphenylessigsäure-n-2-octylamid 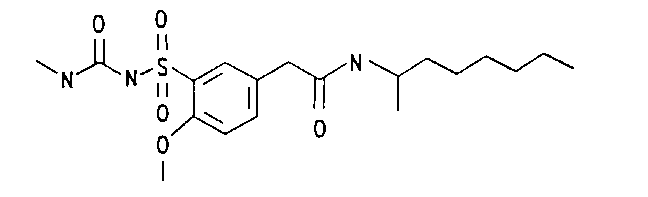 Öl

Beispiel 35: 1-[3-Sulfonylamino-N-(methylaminothiocarbonyl)-(4-methoxyphenyl)]-cyclopropan-1-carbonsäure-n-2-(S)-heptylamid 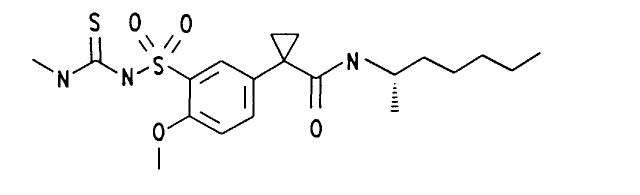 Smp: 184°C

Beispiel 36: 3-Sulfonylamino-N-(ethylaminothiocarbonyl)-4-methoxyphenylessigsäure-n-2-octylamid 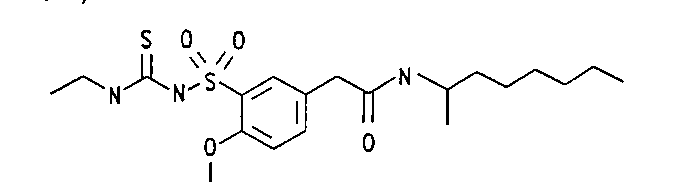 Smp: 125°C

Beispiel 37: 3-Sulfonylamino-N-(isopropylaminothiocarbonyl)-4-methoxyphenylessigsäure-n-2-octylamid 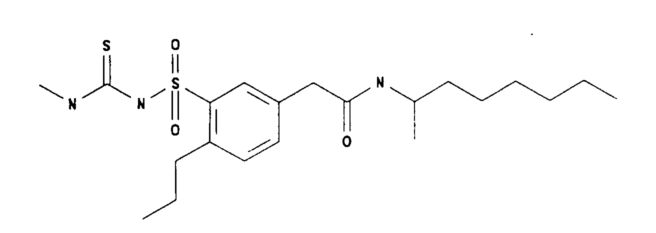 Smp: 141°C

Beispiel 38: 3-Sulfonylamino-N-(cyclopropylaminothiocarbonyl)-4-methoxyphenyl-essigsäure-n-2-octylamid 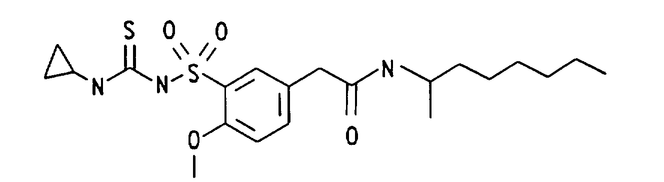 Smp: 128°C

Beispiel 39: 3-Sulfonylamino-N-(cyclohexylaminothiocarbonyl)-4-methoxyphenyl-essigsäure-n-2-octylamid 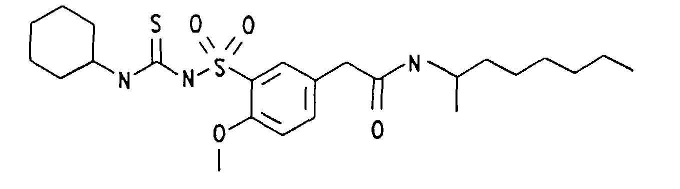 Öl.

Beispiel 40: 3-Sulfonylamino-N-(isopropylaminocarbonyl)-4-methoxyphenylessigsäure-n-2-octylamid 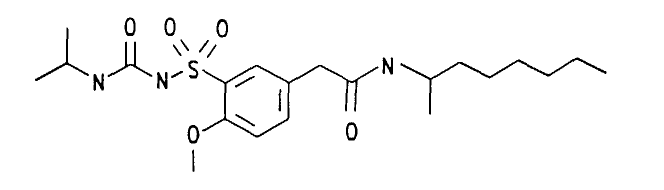 Smp: 118°C

Beispiel 41: 3-Sulfonylamino-N-(cyclopropylaminocarbonyl)-4-methoxyphenylessigsäure-n-2-octylamid 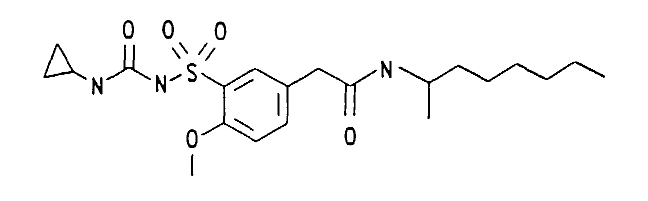 Öl.

Beispiel 42: 3-Sulfonylamino-N-(ethylaminocarbonyl)-4-methoxyphenylessigsäure-n-2-octylamid 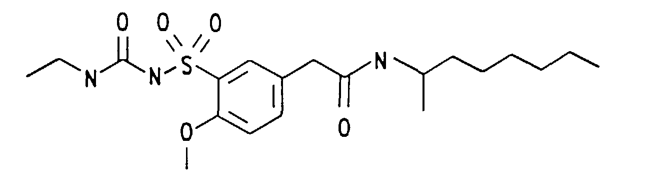 Öl.

Beispiel 43: 3-Sulfonylamino-N-(methylaminocarbonyl)-4-ethoxyphenylessigsäure-n-2-octylamid 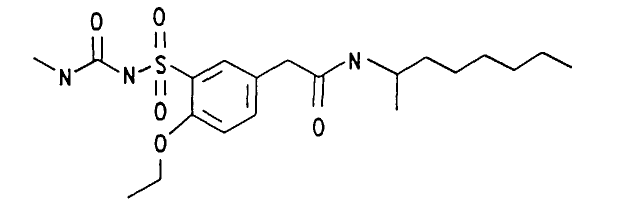 Smp: 78°C

Beispiel 44: 3-Sulfonylamino-N-(isopropylaminothiocarbonyl)-4-methoxyphenylessigsäure-n-2-octylamid 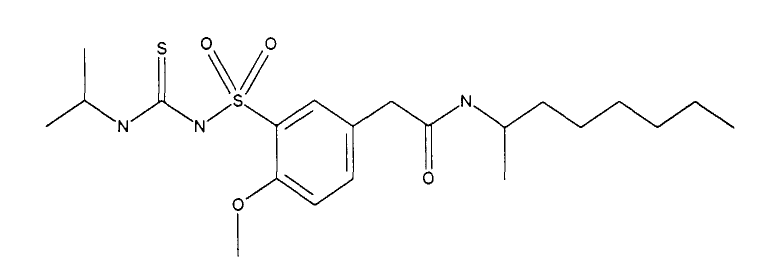 Smp. 141°C.

Beispiel 45: 3-Sulfonylamino-N-(methylaminothiocarbonyl)-4-methoxyphenylessigsäure-n-4-nonylamid 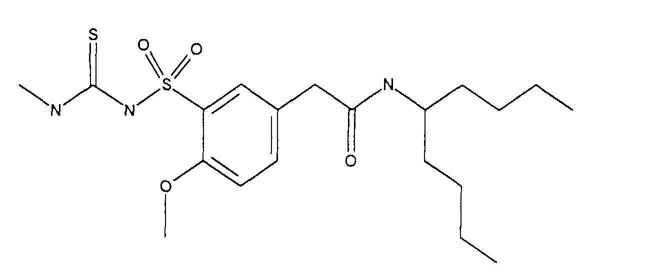 Smp. 145°C.

Beispiel 46: 3-Sulfonylamino-N-(methylaminothiocarbonyl)-4-methoxyphenylessigsäure-n-3-heptylamid 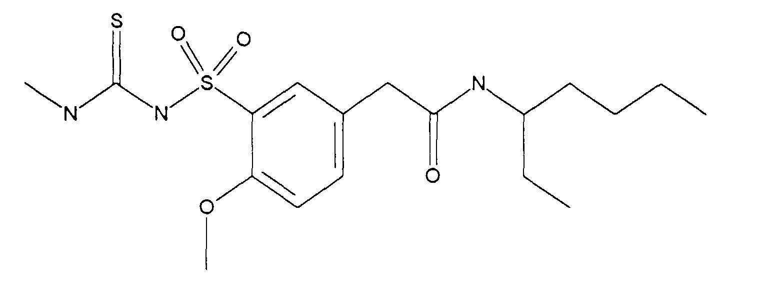 Smp. 100°C

Beispiel 47: 3-Sulfonylamino-N-(methylaminothiocarbonyl)-4-methoxyphenylessigsäure-n-2-nonylamid 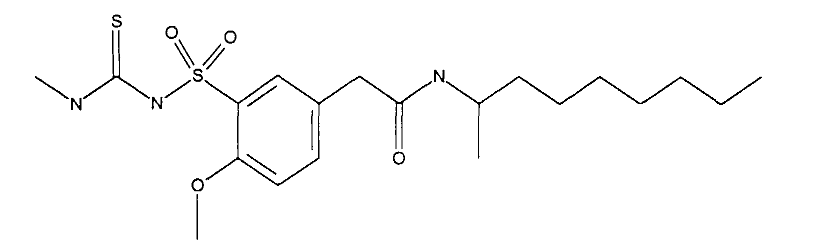 Smp. 107°C

Beispiel 48: 3-Sulfonylamino-N-(ethylaminothiocarbonyl)-4-methoxyphenylessigsäure-n-2-(s)-heptylamid 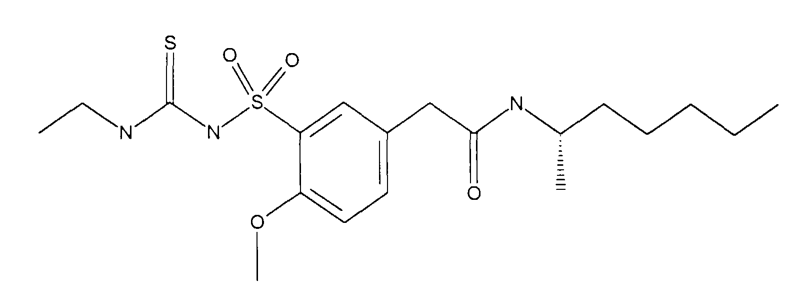 Smp. 145°C

Beispiel 49: 3-Sulfonylamino-N-(isopropylaminothiocarbonyl)-4-methoxyphenylessigsäure-n-2-(s)-heptylamid 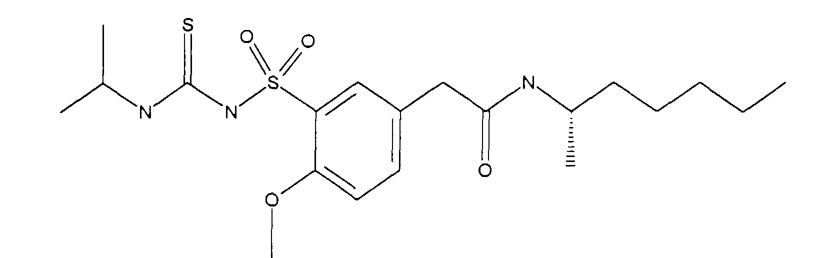 Smp. 159°C

Beispiel 50: 3-Sulfonylamino-N-(tert-butylaminothiocarbonyl)-4-methoxyphenylessigsäure-n-2-octylamid 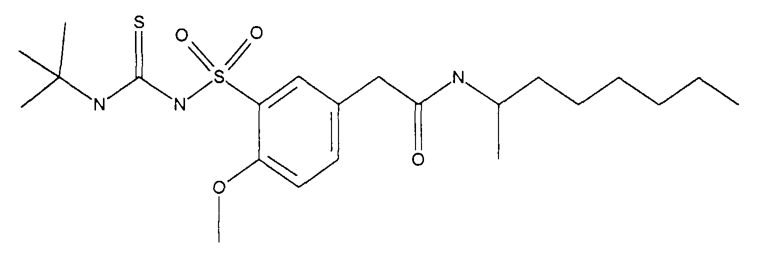 Smp. 126°c

Beispiel 51: 3-Sulfonylamino-N-(methylaminothiocarbonyl)-4-methoxyphenylessigsäure-n-2-decylamid 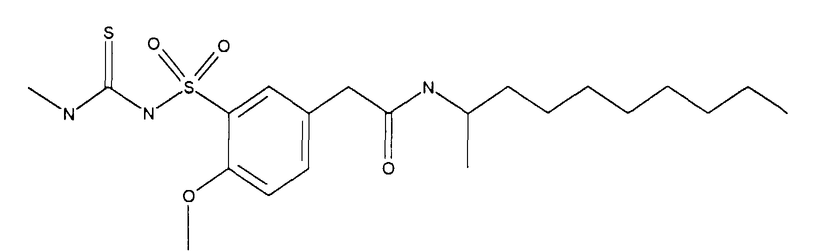 Smp. 112°C

Beispiel 52: 3-Sulfonylamino-N-(cyclopropylaminothiocarbonyl)-4-methoxyphenyl-essigsäure-n-2(s)-heptylamid 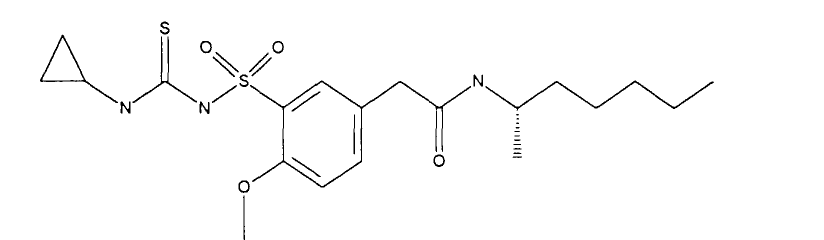 Smp. 165°C

Beispiel 53: 3-Sulfonylamino-N-(methylaminothiocarbonyl)-4-propoxyphenylessigsäure-n-2-(s)-heptylamid 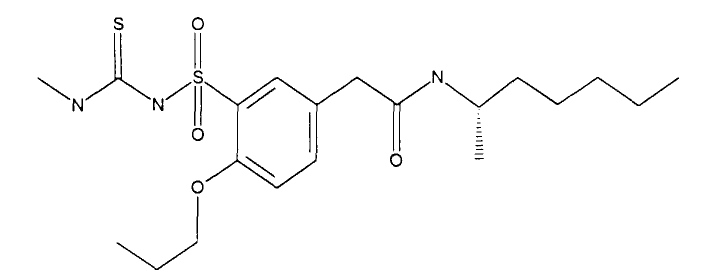 Smp. 120°C

Beispiel 54: 3-Sulfonylamino-N-(methylaminothiocarbonyl)-4-methoxyphenylessigsäure-n-4-nonylamid 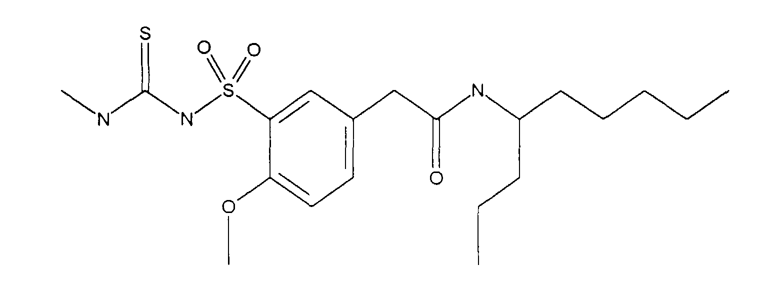 Smp. 122°C

Beispiel 55: 3-Sulfonylamino-N-(methylaminothiocarbonyl)-4-methoxyphenylessigsäure-n-4-nonyl-2-methyl-amid 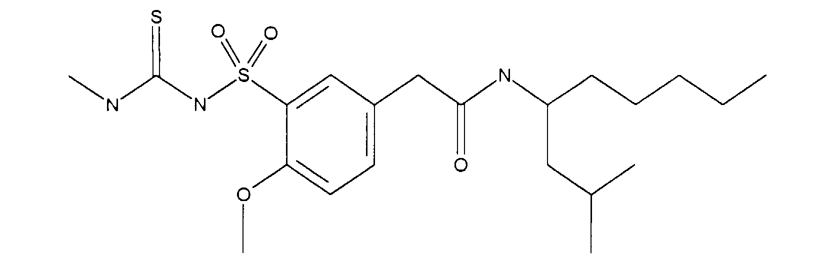 Smp. 136°C

Beispiel 56: 3-Sulfonylamino-N-(methylaminothiocarbonyl)-4-methoxyphenylessigsäure-n-4-nonyl-3-methyl-amid 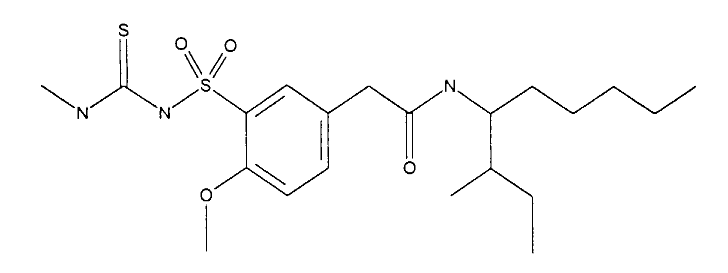 Smp. 129°C

Beispiel 57: 3-Sulfonylamino-N-(methylaminothiocarbonyl)-4-methoxyphenylessigsäure-n-3-octyl-2,2-dimethyl-amid 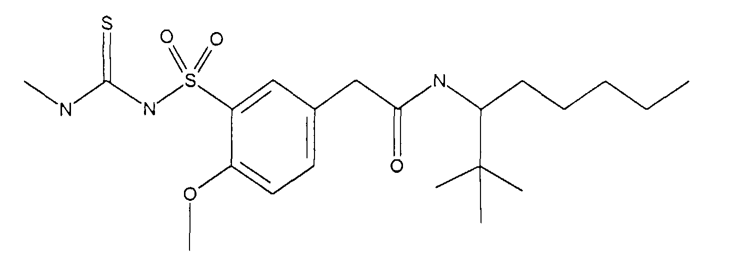 Smp. 178°C

Beispiel 58: 3-Sulfonylamino-N-(methylaminothiocarbonyl)-4-methoxyphenylessigsäure-n-3-octyl-2-methyl-amid 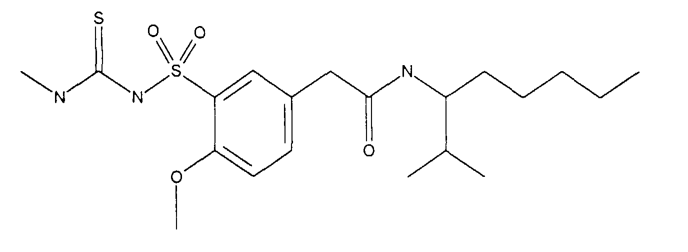 Smp. 123°C

### Pharmakologische Daten:

Mit den folgenden Modellen können die therapeutischen Eigenschaften der Verbindungen I nahegelegt werden:

### (1) Aktionspotentialdauer am Papillarmuskel des Meerschweinchens:

### (a) Einleitung

ATP-Mangelzustände, wie sie während einer Ischämie in der Herzmuskelzelle beobachtet werden, führen zu einer Verkürzung der Aktionspotentialdauer. Sie gelten als einer der Ursachen für sogenannte Reentry-Arrhythmien, die den plötzlichen Herztod verursachen können. Die Öffnung von ATP-sensitiven K-Kanälen durch das Absinken von ATP gilt hierfür als ursächlich.

### (b) Methode

Zur Messung des Aktionspotentials wird eine Standard-Mikroelektroden-Technik eingesetzt. Hierfür werden Meerschweinchen beiderlei Geschlechts durch Schlag auf den Kopf getötet, die Herzen entnommen, die Papillarmuskeln herausgetrennt und in einem Organbad aufgehängt. Das Organbad wird mit Ringerlösung (0.9% NaCl, 0.048% KCl, 0.024% CaCl₂, 0.02% NaHCO₃ und 0.1% Glucose) durchspült und mit einer Mischung aus 95% Sauerstoff und 5% Kohlendioxid bei einer Temperatur von 36°C begast. Der Muskel wird über eine Elektrode mit Rechteck-Impulsen von 1 V und 1 ms Dauer und einer Frequenz von 2 Hz angeregt. Das Aktionspotential wird durch eine intrazellulär eingestochene Glas-Mikroelektrode, die mit 3 mMol KCl-Lösung gefüllt ist, abgeleitet und registriert. Die zu prüfenden Substanzen wurden der Ringerlösung in einer Konzentration von 2.2·10⁻⁵ Mol pro Liter zugesetzt. Das Aktionspotential wird mit einem Amplifier von Hugo Sachs verstärkt und am Oszilloskop dargestellt. Die Dauer des Aktionspotentials wird bei einem Repolarisierungsgrad von 95% (APD95) bestimmt.

Aktionspotentialverkürzungen werden entweder durch Zugabe einer 1 µM starken Lösung des Kaliumkanalöffners Hoe 234 (J. Kaiser, H. Gögelein, Naunyn-Schmiedebergs Arch. Pharm. 1991, 343, R 59) oder durch Zugabe von 2-Desoxyglucose hervorgerufen. Der aktionspotentialverkürzende Effekt dieser Substanzen wurde durch die gleichzeitige Gabe der Testsubstanzen verhindert oder verringert. Testsubstanzen wurden als Stammlösungen in Propandiol der Badlösung zugesetzt. Die angegebenen Werte beziehen sich auf Messungen 30 min nach der Zugabe. Glibenclamid diente in diesen Messungen als Standard. Die Testkonzentration beträgt in allen Fällen 2 x 10⁻⁵ M.

### (c) Resultate:

**Folgende Werte wurden gemessen:.**

| Beispiel Nr. | APD95-Anfang [ms] | APD95-30 min [ms] |
|---|---|---|
| 1 | 150 ± 21 | 157 ± 11 |

## Patentansprüche

1. Substituierte Benzolsulfonylharnstoffe und -thioharnstoffe der Formel I, 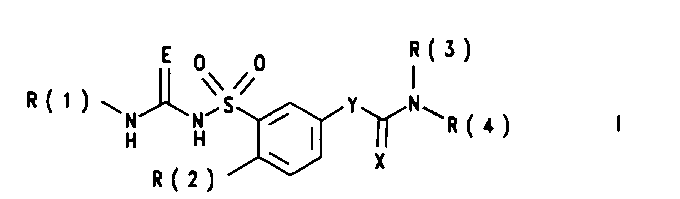 worin bedeuten:
R(1) Wasserstoff, Alkyl mit 1, 2, 3, 4, 5, 6 oder 7 C-Atomen, Cycloalkyl mit 3, 4, 5, 6 oder 7 C-Atomen;
R(2) Wasserstoff, Alkyl mit 1, 2, 3, 4, 5 oder 6 C-Atomen, Cycloalkyl mit 3, 4, 5 oder 6 C-Atomen oder eine (C₁-C₈)-Kette, in der n C-Atome durch Heteroatome, z.B. O, N, S, ersetzt sind ( mit n = 1 - 4), F, Cl;
R(3) H, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. O, N, S, ersetzt sind (n = 1, 2, 3 oder 4);
R(4) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. O, N, S, ersetzt sind (n = 1, 2, 3 oder 4);
oder
R(3) und R(4) gemeinsam einen (CH₂)₂₋₈-Ring bilden, in dem eine oder mehrere der CH₂-Gruppen durch Heteroatome ersetzt sein können;
E Sauerstoff oder Schwefel;
X Sauerstoff oder Schwefel;
Y [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
m 1, 2, 3 oder 4;
sowie deren pharmazeutisch verträgliche Salze.

2. Verbindung der Formel I nach Anspruch 1, dadurch gekennzeichnet, daß darin bedeuten:
R(1) Wasserstoff, Alkyl mit 1, 2, 3, 4, 5, 6 oder 7 C-Atomen, Cycloalkyl mit 3, 4, 5, 6 oder 7 C-Atomen;
R(2) Wasserstoff, F, Cl, Alkyl mit 1, 2, 3, 4, 5 oder 6 C-Atomen, Cycloalkyl mit 3, 4, 5 oder 6 C-Atomen oder eine (C₂-C₇)-Kette, in der 1, 2, 3 oder 4 CH₂-Gruppen durch Heteroatome O, N, oder S ersetzt sind;
R(3) und R(4) gleich oder verschieden Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der 1, 2, 3 oder 4 CH₂-Gruppen durch Heteroatome O, S oder NH ersetzt sind;
oder
R(3) und R(4) gemeinsam (CH₂)₂₋₈, worin eine der CH₂-Gruppen durch ein Heteroatom O, S oder NH ersetzt sein kann;
E Sauerstoff oder Schwefel;
X Sauerstoff oder Schwefel;
Y [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
m 1, 2, 3 oder 4;
sowie deren pharmazeutisch verträgliche Salze.

3. Verbindung der Formel I nach mindestens einem der Ansprüche 1 oder 2, dadurch gekennzeichnet, daß darin bedeuten:
R(1) Wasserstoff, Alkyl mit 1, 2, 3 oder 4 C-Atomen, Cycloalkyl mit 3, 4, 5, 6 oder 7 C-Atomen;
R(2) F, Cl, Alkyl mit 1, 2, 3 oder 4 C-Atomen oder eine (C₂-C₇)-Kette, worin 1, 2, 3 oder 4 CH₂-Gruppen durch Heteroatome O, S oder NH ersetzt sind;
R(3) und R(4) gleich oder verschieden Wasserstoff, Alkyl mit 1, 2, 3, 4, 5, 6, 7, 8, 9 oder 10 C-Atomen, Cycloalkyl mit 3, 4, 5 oder 6 C-Atomen oder eine (C₁-C₁₀)-Kette, worin 1, 2, 3 oder 4 CH₂-Gruppen durch Heteroatome O, S, NH ersetzt sind;
oder
R(3) und R(4) gemeinsam (CH₂)₂₋₇, worin eine der CH₂-Gruppen durch Heteroatome O, S, NH ersetzt sein kann;
E Sauerstoff oder Schwefel;
X Sauerstoff oder Schwefel;
Y [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
m 1, 2, 3 oder 4;
sowie deren pharmazeutisch verträgliche Salze.

4. Verbindungen der Formel I nach Anspruch 1, dadurch gekennzeichnet, daß darin bedeuten:
R(1) Wasserstoff, Alkyl mit 1, 2, 3, 4, 5, 6 oder 7 C-Atomen, Cycloalkyl mit 3, 4, 5, 6 oder 7 C-Atomen;
R(2) Alkoxy mit 1, 2, 3, 4, 5 oder 6 C-Atomen oder Mercaptoalkyl mit 1, 2, 3, 4, 5 oder 6 C-Atomen;
R(3) H, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. O, N, S, ersetzt sind (n = 1, 2, 3 oder 4);
R(4) Wasserstoff, Alkyl mit 1, 2, 3, 4, 5, 6, 7, 8, 9, 10, 11 oder 12 C-Atomen, Cycloalkyl mit 3, 4, 5, 6, 7, 8, 9 oder 10 C-Atomen, Fluoralkyl mit 1, 2, 3, 4, 5 oder 6 C-Atomen, Fluorcycloalkyl mit 3, 4, 5 oder 6 C-Atomen oder eine (C₁-C₁₀)-Kette, in der n C-Atome durch Heteroatome, z.B. O, N, S, ersetzt sind (n = 1, 2, 3 oder 4);
oder
R(3) und R(4) gemeinsam einen (CH₂)₂₋₈-Ring bilden, in dem eine oder mehrere der CH₂-Gruppen durch Heteroatome ersetzt sein können;
E Sauerstoff oder Schwefel;
X Sauerstoff oder Schwefel;
Y [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
m 1, 2, 3 oder 4;
sowie deren pharmazeutisch verträgliche Salze.

5. Verbindung der Formel I nach mindestens einem der Ansprüche 1 bis 4, dadurch gekennzeichnet, daß darin bedeuten:
R(1) Wasserstoff, Alkyl mit 1, 2, 3 oder 4 C-Atomen, Cycloalkyl mit 3 oder 4 C-Atomen;
R(2) Methoxy oder Ethoxy;
R(3) und R(4) gleich oder verschieden Wasserstoff, Alkyl mit 3, 4, 5, 6, 7 oder 8 C-Atomen, Cycloalkyl mit 3, 4, 5 oder 6 C-Atomen oder eine (C₃₋₈H₇₋₁₇)-Gruppe, worin 1, 2, 3 oder 4 CH₂-Gruppen durch Heteroatome 0, S, NH ersetzt sind;
oder
R(3) und R(4) gemeinsam eine (CH₂)₂₋₈-Gruppe, worin eine der CH₂-Gruppen durch Heteroatome O, S, NH ersetzt sein kann;
E Schwefel oder Sauerstoff;
X Sauerstoff;
Y [CR(5)R(5')]ₘ;
R(5) und R(5')
unabhängig voneinander Wasserstoff oder Alkyl mit 1 oder 2 C-Atomen;
wobei die Glieder [CR(5)R(5')] gleich oder verschieden sind;
m 1 oder 2;
sowie deren pharmazeutisch verträgliche Salze.

6. Verbindung der Formel I nach mindestens einem der Ansprüche 1 bis 5, dadurch gekennzeichnet, daß darin bedeuten:
R(1) Wasserstoff, Alkyl mit 1, 2 oder 3 C-Atomen oder Cycloalkyl mit 3 C-Atomen;
R(2) Methoxy oder Ethoxy;
R(3) und R(4) gleich oder verschieden Wasserstoff, Alkyl mit 5, 6, 7 oder 8 C-Atomen, Cycloalkyl mit 5 oder 6 C-Atomen oder eine (C₅₋₈H₁₁₋₁₇)-Gruppe, worin 1, 2, 3 oder 4 der CH₂-Gruppen durch Heteroatome O, S, NH ersetzt sind;
oder
R(3) und R(4) gemeinsam (CH₂)₅₋₈, worin eine der CH₂-Gruppen durch Heteroatome O, S, NH ersetzt sein kann;
E Sauerstoff oder Schwefel;
X Sauerstoff;
Y [CR(5)R(5')]ₘ;
R(5) und R(5')
unabhängig voneinander Wasserstoff oder Methyl, wobei die Glieder [CR(5)R(5')] gleich oder verschieden sind;
m 1 oder 2;
sowie deren pharmazeutisch verträgliche Salze.

7. Verbindung der Formel I nach mindestens einem der Ansprüche 1 bis 6, dadurch gekennzeichnet, daß darin bedeuten:
R(1) Wasserstoff, Alkyl mit 1, 2 oder 3 C-Atomen oder Cycloalkyl mit 3 C-Atomen;
R(2) Methoxy oder Ethoxy;
R(3) Wasserstoff;
R(4) Alkyl mit 5, 6, 7 oder 8 C-Atomen, Cycloalkyl mit 5 oder 6 C-Atomen oder eine (C₅₋₈H₁₁₋₁₇)-Gruppe, in der 1, 2, 3 oder 4 CH₂-Gruppen durch Heteroatome O, S, NH ersetzt sind;
E Schwefel oder Sauerstoff;
X Sauerstoff;
Y [CR(5)R(5')]ₘ;
R(5) und R(5')
unabhängig voneinander Wasserstoff oder Methyl;
wobei die Glieder [CR(5)R(5')] gleich oder verschieden sind;
m 1 oder 2;
sowie deren pharmazeutisch verträgliche Salze.

8. Substituierter Benzolsulfonylthioharnstoff der Formel 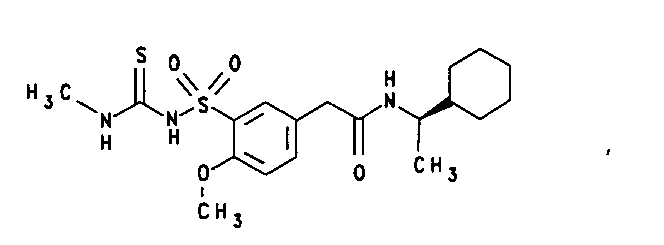 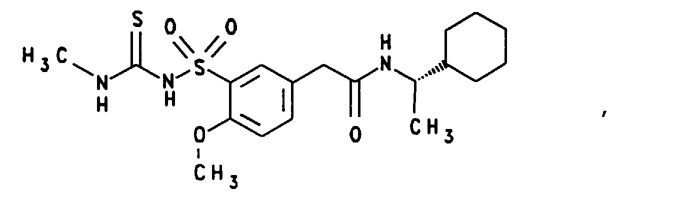 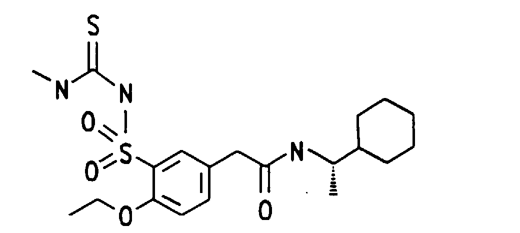 oder 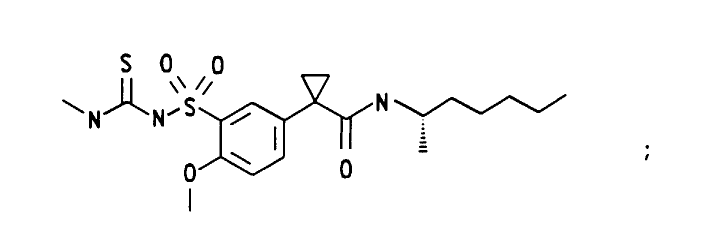 sowie seine pharmazeutisch verträgliche Salze.

9. Verfahren zur Herstellung einer Verbindung der Formel I nach Anspruch 1, dadurch gekennzeichnet, daß man
(a) ein Sulfonamid der Formel II 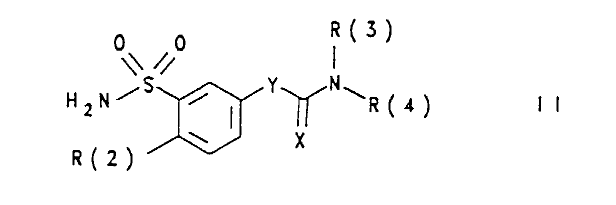 oder dessen Salz der Formel III 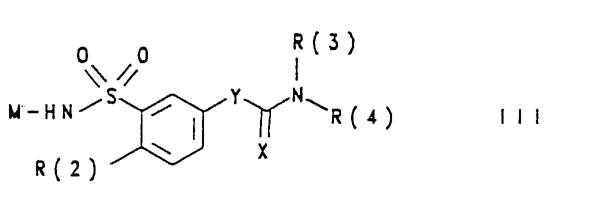 worin R(2), R(3), R(4), X und Y die in Anspruch 1 angegebenen Bedeutungen haben und das Kation M für ein Alkali- oder Erdalkaliion steht, mit einem R(1)-substituierten Isocyanat der Formel IV
R(1) - N = C = O IV,
worin R(1) die in Anspruch 1 angegebene Bedeutung hat, zum substituierten Benzolsulfonylharnstoff I umsetzt; 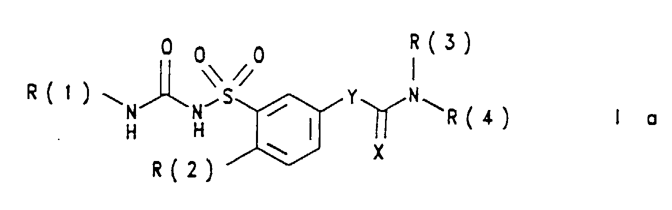 oder daß man
(b) einen Benzolsulfonylharnstoff der Formel I a aus einem aromatischen Benzolsulfonamid II oder dessen Salz III mit einem R(1)-substituierten Trichloracetamid der Formel V
Cl₃C-C(=O)-NH-R(1) V
in Gegenwart einer Base herstellt; oder daß man
(c) einen Benzolsulfonylthioharnstoff I b 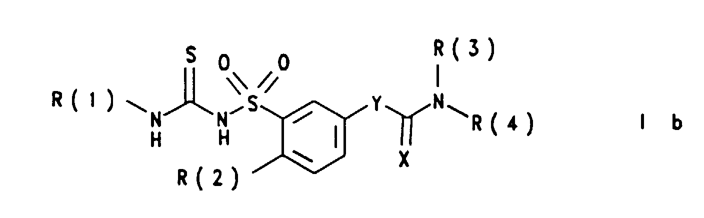 aus einem Benzolsulfonamid II oder dessen Salz III und einem R(1)-substituierten Thioisocyanat VI
R(1) - N = C = S VI
herstellt; oder daß man
(d) einen Benzolsulfonylharnstoff der Formel I a durch eine Umwandlungsreaktion aus einem Benzolsulfonylthioharnstoff der Formel I b darstellt;
oder daß man
(e) einen Benzolsulfonylharnstoff I durch Umsetzung eines Amins der Formel R(1)-NH₂ mit einem Benzolsulfonylisocyanat der Formel VII 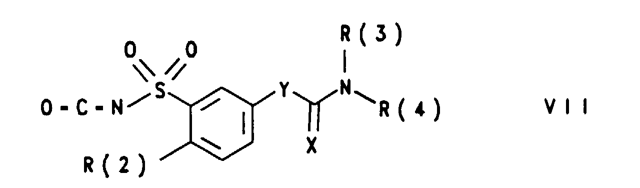 herstellt;
oder daß man
(f) einen Benzolsulfonylthioharnstoff I b durch Umsetzung eines Amins der Formel R(1)-NH₂ mit einem Benzolsulfonylisothiocyanat der Formel VIII 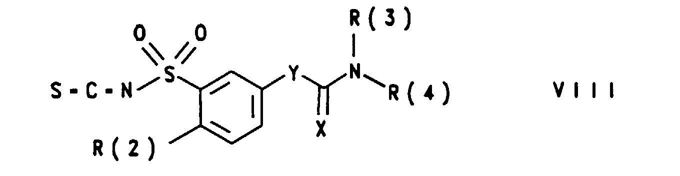 herstellt;
oder daß man
(g) einen Benzolsulfonylharnstoff I a aus einem Benzolsulfonylharnstoff der Formel IX 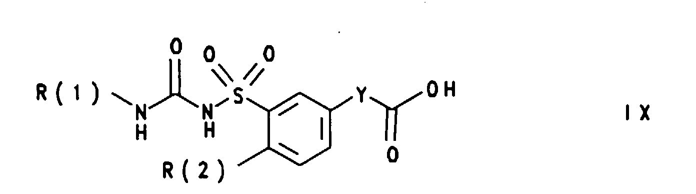 und R(3)R(4)NH mittels wasserentziehender Mittel oder Aktivierung mittels Carbonsäurehalogeniden bzw. Bildung gemischter Anhydride darstellt;
oder daß man
(h) einen Benzolsulfonylthioharnstoff I b aus einem Benzolsulfonylthioharnstoff der Formel X 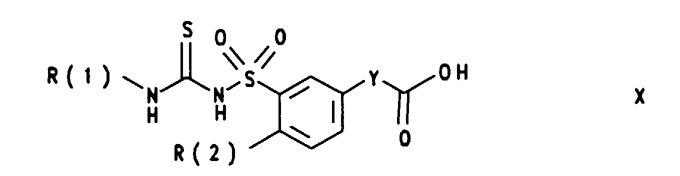 und R(3)R(4)NH mittels wasserentziehender Mittel oder Aktivierung mittels Carbonsäurehalogenide bzw. Bildung gemischter Anhydride dargestellt.

10. Verwendung einer Verbindung der Formel I nach einem oder mehreren der Ansprüche 1 bis 8 oder eines pharmazeutisch verträglichen Salzes davon zur Herstellung eines Medikaments zur Behandlung von Herzrhythmusstörungen.

11. Verwendung einer Verbindung der Formel I nach einem oder mehreren der Ansprüche 1 bis 8 oder eines pharmazeutisch verträglichen Salzes davon zur Herstellung eines Medikaments zur Verhinderung des plötzlichen Herztods.

12. Verwendung einer Verbindung der Formel I nach einem oder mehreren der Ansprüche 1 bis 8 oder eines pharmazeutisch verträglichen Salzes davon zur Herstellung eines Medikaments zur Behandlung von ischämischen Zuständen des Herzens.

13. Verwendung einer Verbindung der Formel I nach einem oder mehreren der Ansprüche 1 bis 8 oder eines pharmazeutisch verträglichen Salzes davon zur Herstellung eines Medikaments zur Behandlung der geschwächten Herzkraft.

14. Verwendung einer Verbindung der Formel I nach einem oder mehreren der Ansprüche 1 bis 8 oder eines pharmazeutisch verträglichen Salzes davon zur Herstellung eines Medikaments zur Verbesserung der Herzfunktion nach Herztransplantation.

15. Heilmittel, gekennzeichnet durch eine wirksame Menge einer Verbindung der Formel I nach einem oder mehreren der Ansprüche 1 bis 8 oder eines pharmazeutisch verträglichen Salzes davon.

## Claims

1. A substituted benzenesulfonylurea or -thiourea of the formula I 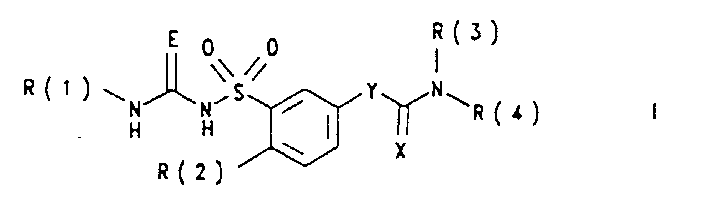 in which:
R(1) is hydrogen, alkyl having 1, 2, 3, 4, 5, 6 or 7 carbon atoms or cycloalkyl having 3, 4, 5, 6 or 7 carbon atoms;
R(2) is hydrogen, alkyl having 1, 2, 3, 4, 5 or 6 carbon atoms, cycloalkyl having 3, 4, 5 or 6 carbon atoms or a (C₁-C₈) chain in which n carbon atoms are replaced by heteroatoms, e.g. O, N and S (where n = 1-4), F or Cl:
R(3) is H, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (C₁-C₁₀) chain in which n carbon atoms are replaced by heteroatoms, e.g. O, N or S (n = 1, 2, 3 or 4);
R(4) is hydrogen, alkyl having 1, 2, 3, 4, 5, 6, 7, 8, 9, 10, 11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (C₁-C₁₀) chain in which n carbon atoms are replaced by heteroatoms, e.g. O, N or S (n = 1, 2, 3 or 4);
or
R(3) and R(4) together form a (CH₂)₂₋₈ ring, in which one or more of the CH₂ groups can be replaced by heteroatoms;
E is oxygen or sulfur;
X is oxygen or sulfur;
Y is [CR(5)R(5')]ₘ;
R(5) and R(5')
independently of one another are hydrogen or alkyl having 1 or 2 carbon atoms;
where the members [CR(5)R(5')] are identical or different;
m is 1, 2, 3 or 4;
or its pharmaceutically tolerable salts.

2. A compound of the formula I as claimed in claim 1, wherein:
R(1) is hydrogen, alkyl having 1, 2, 3, 4, 5, 6 or 7 carbon atoms or cycloalkyl having 3, 4, 5, 6 or 7 carbon atoms;
R(2) is hydrogen, F, Cl, alkyl having 1, 2, 3, 4, 5 or 6 carbon atoms, cycloalkyl having 3, 4, 5 or 6 carbon atoms or a (C₂-C₇) chain in which 1, 2, 3 or 4 CH₂ groups are replaced by heteroatoms O, N or S;
R(3) and R(4) are identical or different and are hydrogen, alkyl having 1, 2, 3, 4, 5, 6, 7,8,9,10,11 or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (C₁-C₁₀) chain in which 1, 2, 3 or 4 CH₂ groups are replaced by heteroatoms O, S or NH;
or
R(3) and R(4) together are (CH₂)₂₋₈, in which one of the CH₂ groups can be replaced by a heteroatom O, S or NH;
E is oxygen or sulfur;
X is oxygen or sulfur;
Y is [CR(5)R(5')]ₘ;
R(5) and R(5')
independently of one another are hydrogen or alkyl having 1 or 2 carbon atoms;
where the members [CR(5)R(5')] are identical or different:
m is 1, 2, 3 or 4;
or its pharmaceutically tolerable salts.

3. A compound of the formula I as claimed in at least one of claims 1 and 2, wherein:
R(1) is hydrogen, alkyl having 1, 2, 3 or 4 carbon atoms or cycloalkyl having 3, 4, 5, 6 or 7 carbon atoms;
R(2) is F, Cl, alkyl having 1, 2, 3 or 4 carbon atoms or a (C₂-C₇) chain, in which 1, 2, 3 or 4 CH₂ groups are replaced by heteroatoms O, S or NH;
R(3) and R(4) are identical or different and are hydrogen, alkyl having 1, 2, 3, 4, 5, 6, 7, 8, 9 or 10 carbon atoms, cycloalkyl having 3, 4, 5 or 6 carbon atoms or a (C₁-C₁₀) chain, in which 1, 2, 3 or 4 CH₂ groups are replaced by heteroatoms O, S or NH;
or
R(3) and R(4) are together (CH₂)₂₋₇, in which one of the CH₂ groups can be replaced by heteroatoms O, S or NH;
E is oxygen or sulfur;
X is oxygen or sulfur;
Y is [CR(5)R(5')]ₘ;
R(5) and R(5')
independently of one another are hydrogen or alkyl having 1 or 2 carbon atoms;
where the members [CR(5)R(5')] are identical or different;
m is 1, 2, 3 or 4;
or its pharmaceutically tolerable salts.

4. A compound of the formula I as claimed in Claim 1, wherein:
R(1) is hydrogen, alkyl having 1, 2, 3, 4, 5, 6 or 7 carbon atoms or cycloalkyl having 3, 4, 5, 6 or 7 carbon atoms;
R(2) is alkoxy having 1, 2, 3, 4, 5 or 6 carbon atoms or mercaptoalkyl having 1, 2, 3, 4, 5 or 6 carbon atoms:
R(3) is H, alkyl having 1, 2, 3, 4 ,5, 6, 7, 8, 9, 10, 11 or 12 carbon atoms, cycloalkyl having 3, 4, 5, 6, 7, 8, 9 or 10 carbons atoms, fluoroalkyl having 1, 2, 3, 4, 5 or 6 carbon atoms, fluorocycloalkyl having 3, 4, 5 or 6 carbon atoms or a (C₁-C₁₀) chain, in which n carbon atoms are replaced by heteroatoms, e.g. O, N or S (n=1, 2, 3 or 4);
R(4) is hydrogen, alkyl having 1, 2, 3, 4, 5, 6, 7, 8, 9, 10, 11, or 12 carbon atoms, cycloalkyl having 3, 4, 5, 6, 7, 8, 9 or 10 carbon atoms, fluoroalkyl having 1, 2, 3, 4, 5 or 6 carbon atoms, fluorocycloalkyl having 3, 4, 5 or 6 carbon atoms or a (C₁-C₁₀) chain, in which n carbon atoms are replaced by heteroatoms, e.g. O, N or S (n=1, 2, 3 or 4)
or
R(3) and R(4) together form a (CH₂)₂₋₈ ring, in which one or more of the CH₂ groups can be replaced by heteroatoms;
E is oxygen or sulfur;
X is oxygen or sulfur;
Y is [CR(5)R(5')]ₘ;
R(5) and R(5')
independently of one another are hydrogen or alkyl having 1 or 2 carbon atoms;
where the members [CR(5)R(5')] are identical or different;
m is 1, 2, 3 or 4;
or its pharmaceutically tolerable salts.

5. A compound of the formula I as claimed in at least one of claims 1 to 4, wherein:
R(1) is hydrogen, alkyl having 1, 2, 3 or 4 carbon atoms or cycloalkyl having 3 or 4 carbon atoms;
R(2) is methoxy or ethoxy;
R(3) and R(4) are identical or different and are hydrogen, alkyl having 3, 4, 5, 6, 7 or 8 carbon atoms, cycloalkyl having 3, 4, 5 or 6 carbon atoms or a (C₃₋₈H₇₋₁₇) group, in which 1, 2, 3 or 4 CH₂ groups are replaced by heteroatoms O, S or NH;
or
R(3) and R(4) together are a (CH₂)₂₋₈ group, in which one of the CH₂ groups can be replaced by heteroatoms O, S or NH;
E is sulfur or oxygen;
X is oxygen;
Y is [CR(5)R(5')]ₘ;
R(5) and R(5')
independently of one another are hydrogen or alkyl having 1 or 2 carbon atoms;
where the members [CR(5)R(5')] are identical or different;
m is 1 or 2;
or its pharmaceutically tolerable salts.

6. A compound of the formula I as claimed in at least one of claims 1 to 5, wherein:
R(1) is hydrogen, alkyl having 1, 2 or 3 carbon atoms or cycloalkyl having 3 carbon atoms;
R(2) is methoxy or ethoxy;
R(3) and R(4) are identical or different and are hydrogen, alkyl having 5, 6, 7 or 8 carbon atoms, cycloalkyl having 5 or 6 carbon atoms or a (C₅₋₈H₁₁₋₁₇) group, in which 1, 2, 3 or 4 CH₂ groups are replaced by heteroatoms O, S or NH;
or
R(3) and R(4) together are (CH₂)₅₋₈, in which one of the CH₂ groups can be replaced by heteroatoms O, S or NH;
E is oxgyen or sulfur;
X is oxygen;
Y is [CR(5)R(5')]ₘ;
R(5) and R(5')
independently of one another are hydrogen or methyl;
where the members [CR(5)R(5')] are identical or different;
m is 1 or 2;
or its pharmaceutically tolerable salts.

7. A compound of the formula I as claimed in at least one of claims 1 to 6, wherein:
R(1) is hydrogen, alkyl having 1, 2 or 3 carbon atoms or cycloalkyl having 3 carbon atoms;
R(2) is methoxy or ethoxy;
R(3) is hydrogen;
R(4) is alkyl having 5, 6, 7 or 8 carbon atoms, cycloalkyl having 5 or 6 carbon atoms or a (C₅₋₈H₁₁₋₁₇) group, in which 1, 2, 3 or 4 CH₂ groups are replaced by heteroatoms O, S or NH;
E is sulfur or oxygen;
X is oxygen;
Y is [CR(5)R(5')]ₘ;
R(5) and R(5') independently of one another are hydrogen or methyl; where the members [CR(5)R(5')] are identical or different;
m is 1 or 2;
or its pharmaceutically tolerable salts.

8. A substituted benzenesulfonylthiourea of the formula 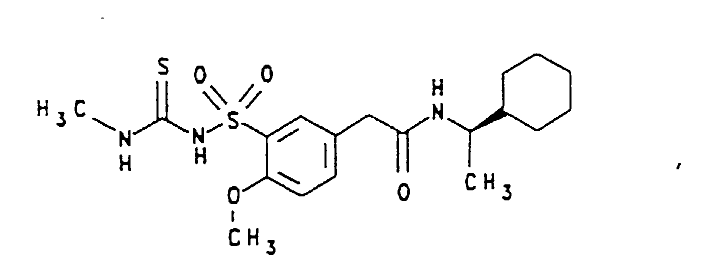 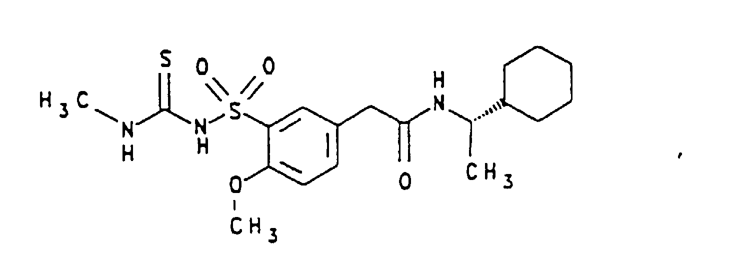 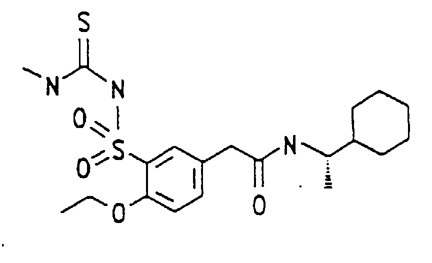 or 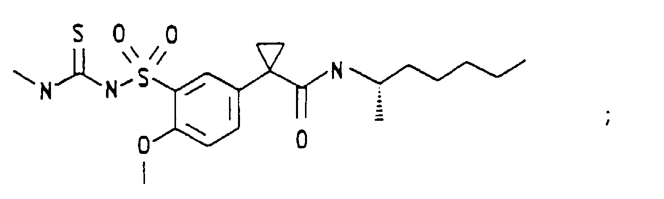 or its pharmaceutically tolerable salts.

9. A process for the preparation of a compound of the formula I as claimed in claim 1, which comprises
(a) reacting a sulfonamide of the formula II 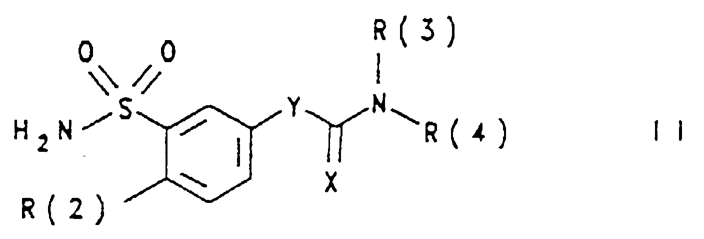 or its salt of the formula III 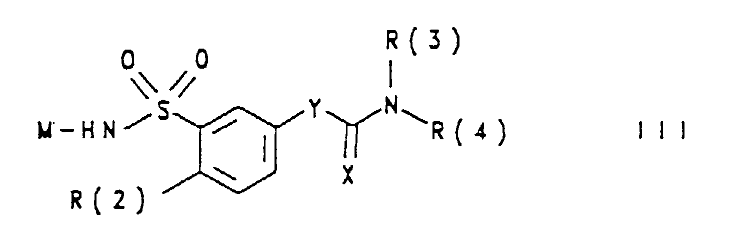 in which R(2), R(3), R(4), X and Y have the meanings indicated in claim 1, and the cation M is an alkali metal or alkaline earth metal ion, with an R(1 )-substituted isocyanate of the formula IV
R(1)-N=C=O IV,
in which R(1) has the meaning indicated in claim 1, to give the substituted benzenesulfonylurea I a; 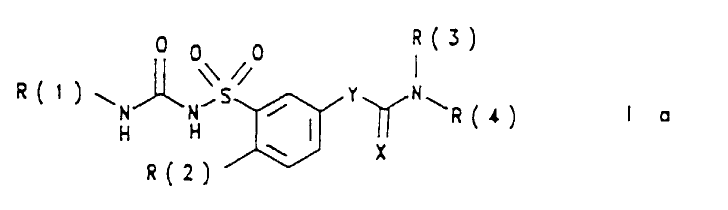 or
(b) preparing a benzenesulfonylurea of the formula I a from an aromatic benzenesulfonamide II or its salt III II using an R(1)-substituted trichloroacetamide of the formula V
Cl₃C-C(=O)-NH-R(1) V
in the presence of a base; or
(c) preparing a benzenesulfonylthiourea I b 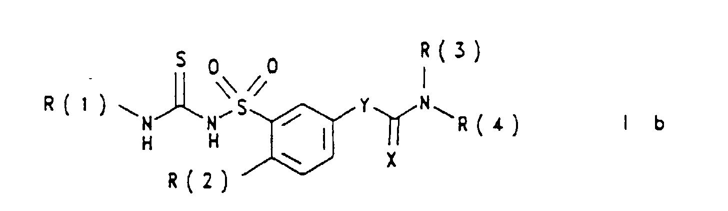 from a benzenesulfonamide II or its salt III and an R(1)-substituted isothiocyanate VI
R(1)-N=C=S VI;
or
(d) preparing a benzenesulfonylurea of the formula I a by a conversion reaction of a benzenesulfonylthiourea of the formula I b; or
(e) preparing a benzenesulfonylurea I a by reaction of an amine of the formula R(1)-NH₂ with a benzenesulfonyl isocyanate of the formula VII 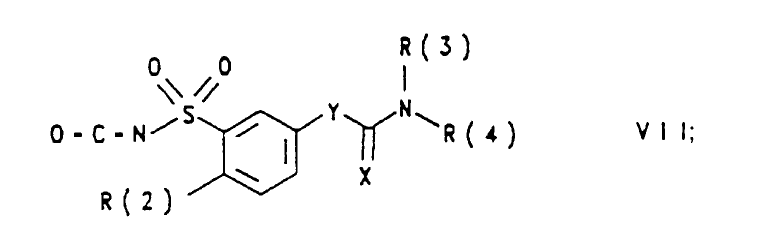 or
(f) preparing a benzenesulfonylthiourea I b by reaction of an amine of the formula R(1)-NH₂ with a benzenesulfonyl isothiocyanate of the formula VIII 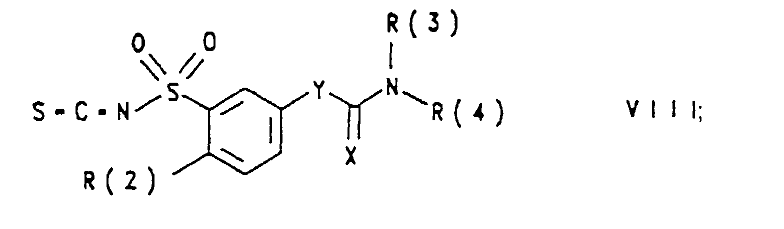 or
(g) preparing a benzenesulfonylurea I a from a benzenesulfonylurea of the formula IX 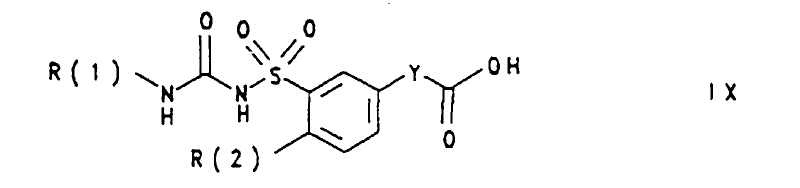 and R(3)R(4)NH by means of dehydrating agents or activation by means of carbonyl halides or formation of mixed anhydrides; or
(h) preparing a benzenesulfonylthiourea I b from a benzenesulfonylthiourea of the formula X 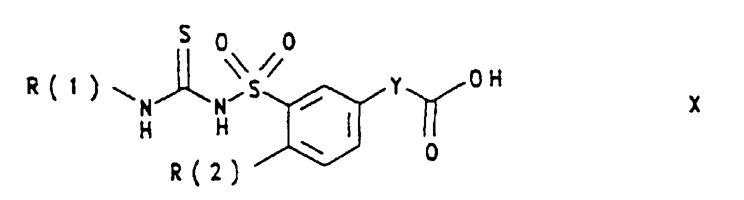 and R(3)R(4)NH by means of dehydrating agents or by activation by means of carbonyl halides or formation of mixed anhydrides.

10. The use of a compound of the formula I as claimed in one or more of claims 1 to 8 or of a pharmaceutically tolerable salt thereof for the production of a medicament for the treatment of cardiac arrhythmias.

11. The use of a compound of the formula I as claimed in one or more of claims 1 to 8 or of a pharmaceutically tolerable salt thereof for the production of a medicament for the prevention of sudden heart death.

12. The use of a compound of the formula I as claimed in one or more of claims 1 to 8 or of a pharmaceutically tolerable salt thereof for the production of a medicament for the treatment of ischemic conditions of the heart.

13. The use of a compound of the formula I as claimed in one or more of claims 1 to 8 or of a pharmaceutically tolerable salt thereof for the production of a medicament for the treatment of weakened cardiac power.

14. The use of a compound of the formula I as claimed in one or more of claims 1 to 8 or of a pharmaceutically tolerable salt thereof for the production of a medicament for the improvement of heart function after heart transplantation.

15. A medicament comprising an effective amount of a compound of the formula I as claimed in one or more of claims 1 to 8 or of a pharmaceutically tolerable salt thereof.

## Revendications

1. Benzènesulfonylurées et -thio-urées substituées de formule I 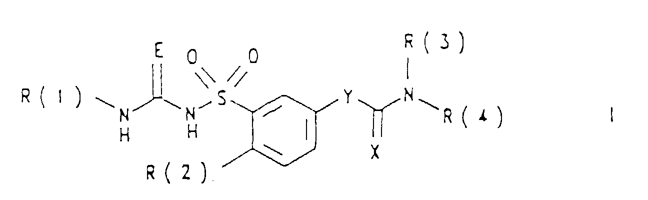 dans laquelle
R(1) représente un atome d'hydrogène ou un groupe alkyle ayant 1, 2, 3, 4, 5, 6 ou 7 atomes de carbone, ou cycloalkyle ayant 3, 4, 5, 6 ou 7 atomes de carbone;
R(2) représente un atome d'hydrogène ou de F ou Cl, ou un groupe alkyle ayant 1, 2, 3, 4, 5 ou 6 atomes de carbone, cycloalkyle ayant 3, 4, 5 ou 6 atomes de carbone ou une chaîne en C₁-C₈ dans laquelle n atomes de carbone sont remplacés par des hétéroatomes, par exemple O, N, S (avec n = 1-4);
R(3) représente H ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou une chaîne en C₁-C₁₀ dans laquelle n atomes de carbone sont remplacés par des hétéroatomes, par exemple O, N, S (n = 1, 2, 3 ou 4);
R(4) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou une chaîne en C₁-C₁₀ dans laquelle n atomes de carbone sont remplacés par des hétéroatomes, par exemple O, N, S (n = 1, 2, 3 ou 4);
ou
R(3) et R(4) forment ensemble un cycle (CH₂)₂₋₈, dans lequel un ou plusieurs des groupes CH₂ peut(peuvent) être remplacé(s) par des hétéroatomes;
E représente un atome d'oxygène ou de soufre;
X représente un atome d'oxygène ou de soufre;
Y représente un groupe [CR(5)R(5/)]ₘ,
R(5) et R(5') représentant, indépendamment l'un de l'autre, un atome d'hydrogène ou un groupe alkyle ayant 1 ou 2 atomes de carbone, les chaînons [CR(5)R(5')] étant identiques ou différents;
m étant 1, 2, 3 ou 4;
ainsi que leurs sels pharmaceutiquement acceptables.

2. Composé de formule I selon la revendication 1, caractérisé en ce que, dans ce composé:
R(1) représente un atome d'hydrogène ou un groupe alkyle ayant 1, 2, 3, 4, 5, 6 ou 7 atomes de carbone, ou cycloalkyle ayant 3, 4, 5, 6 ou 7 atomes de carbone;
R(2) représente un atome d'hydrogène, F, Cl, un groupe alkyle ayant 1, 2, 3, 4, 5 ou 6 atomes de carbone, cycloalkyle ayant 3, 4, 5 ou 6 atomes de carbone ou une chaîne en C₂-C₇, dans laquelle 1, 2, 3 ou 4 groupes CH₂ sont remplacés par les hétéroatomes O, N ou S;
R(3) et R(4) identiques ou différents, représentent un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou une chaîne en C₁-C₁₀ dans laquelle 1, 2, 3 ou 4 groupes CH₂ sont remplacés par des hétéroatomes O ou S ou par NH;
ou
R(3) et R(4) forment ensemble un cycle (XH₂)₂₋₈, dans lequel l'un des groupes CH₂ peut être remplacé par un hétéroatome O, S ou par NH;
E représente un atome d'oxygène ou de soufre;
X représente un atome d'oxygène ou de soufre;
Y représente un groupe [CR(5)R(5')]ₘ,
R(5) et R(5') représentant, indépendamment l'un de l'autre, un atome d'hydrogène ou un groupe alkyle ayant 1 ou 2 atomes de carbone, les chaînons [CR(5)R(5')] étant identiques ou différents;
m étant 1, 2, 3 ou 4;
et sels pharmaceutiquement acceptables d'un tel composé.

3. Composé de formule I selon au moins l'une des revendications 1 et 2, caractérisé en ce que, dans ce composé:
R(1) représente un atome d'hydrogène ou un groupe alkyle ayant 1, 2, 3 ou 4 atomes de carbone, ou cycloalkyle ayant 3, 4, 5, 6 ou 7 atomes de carbone;
R(2) représente F, Cl, un groupe alkyle ayant 1, 2, 3 ou 4 atomes de carbone, ou une chaîne en C₂-C₇, dans laquelle 1, 2, 3 ou 4 groupes CH₂ sont remplacés par les hétéroatomes O, S ou par NH;
R(3) et R(4) identiques ou différents, représentent un atome d'hydrogène ou un groupe alkyle ayant 1, 2, 3, 4, 5, 6, 7, 8, 9 ou 10 atomes de carbone, cycloalkyle ayant 3, 4, 5 ou 6 atomes de carbone, ou une chaîne en C₁-C₁₀ dans laquelle 1, 2, 3 ou 4 groupes CH₂ sont remplacés par des hétéroatomes O ou S ou par NH;
ou
R(3) et R(4) forment ensemble un cycle (CH₂)₂₋₇, dans lequel l'un des groupes CH₂ peut être remplacé par un hétéroatome O, S ou par NH;
E représente un atome d'oxygène ou de soufre;
X représente un atome d'oxygène ou de soufre;
Y représente un groupe [CR(5)R(5')]ₘ,
R(5) et R(5') représentant, indépendamment l'un de l'autre, un atome d'hydrogène ou un groupe alkyle ayant 1 ou 2 atomes de carbone, les chaînons [CR(5)R(5')] étant identiques ou différents;
m étant 1, 2, 3 ou 4;
et sels pharmaceutiquement acceptables d'un tel composé.

4. Composés de formule I selon la revendication 1, caractérisés en ce que, dans ces composés:
R(1) représente un atome d'hydrogène ou un groupe alkyle ayant 1, 2, 3, 4, 5, 6 ou 7 atomes de carbone, ou cycloalkyle ayant 3, 4, 5, 6 ou 7 atomes de carbone;
R(2) représente un groupe alcoxy ayant 1, 2, 3, 4, 5 ou 6 atomes de carbone, ou mercaptoalkyle ayant 1, 2, 3, 4, 5 ou 6 atomes de carbone;
R(3) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou une chaîne en C₁-C₁₀ dans laquelle n atomes de carbone sont remplacés par des hétéroatomes, par exemple O, N, S (n = 1, 2, 3 ou 4);
R(4) représente un atome d'hydrogène ou un groupe alkyle ayant 1, 2, 3, 4, 5, 6, 7, 8, 9, 10, 11 ou 12 atomes de carbone, cycloalkyle ayant 3, 4, 5, 6, 7, 8, 9 ou 10 atomes de carbone, fluoroalkyle ayant 1, 2, 3, 4, 5 ou 6 atomes de carbone, fluorocycloalkyle ayant 3, 4, 5 ou 6 atomes de carbone, ou une chaîne en C₁-C₁₀ dans laquelle n atomes de carbone sont remplacés par des hétéroatomes, par exemple O, N, S (n = 1, 2, 3 ou 4) ;
ou
R(3) et R(4) forment ensemble un cycle (CH₂)₂₋₈, dans lequel un ou plusieurs des groupes CH₂ peut(peuvent) être remplacé(s) par des hétéroatomes;
E représente un atome d'oxygène ou de soufre;
X représente un atome d'oxygène ou de soufre;
Y représente un groupe [CR(5)R(5')]ₘ,
R(5) et R(5') représentant, indépendamment l'un de l'autre, un atome d'hydrogène ou un groupe alkyle ayant 1 ou 2 atomes de carbone, les chaînons [CR(5)R(5')] étant identiques ou différents;
m étant 1, 2, 3 ou 4;
ainsi que leurs sels pharmaceutiquement acceptables.

5. Composé de formule I selon au moins l'une des revendications 1 à 4, caractérisé en ce que, dans ce composé:
R(1) représente un atome d'hydrogène ou un groupe alkyle ayant 1, 2, 3 ou 4 atomes de carbone, ou cycloalkyle ayant 3 ou 4 atomes de carbone;
R(2) représente le groupe méthoxy ou éthoxy;
R(3) et R(4) identiques ou différents, représentent un atome d'hydrogène ou un groupe alkyle ayant 3, 4, 5, 6, 7 ou 8 atomes de carbone, cycloalkyle ayant 3, 4, 5 ou 6 atomes de carbone, ou un groupe (C₃₋₈H₇₋₁₇), dans lequel 1, 2, 3 ou 4 groupes CH₂ sont remplacés par des hétéroatomes O ou S ou par NH;
ou
R(3) et R(4) forment ensemble un groupe (CH₂)₂₋₈, dans lequel l'un des groupes CH₂ peut être remplacé par un hétéroatome O, S ou par NH;
E représente un atome d'oxygène ou de soufre;
X représente un atome d'oxygène;
Y représente un groupe [CR(5)R(5')]ₘ,
R(5) et R(5') représentant, indépendamment l'un de l'autre, un atome d'hydrogène ou un groupe alkyle ayant 1 ou 2 atomes de carbone, les chaînons [CR(5)R(5')] étant identiques ou différents;
m étant 1 ou 2;
et sels pharmaceutiquement acceptables d'un tel composé.

6. Composé de formule I selon au moins l'une des revendications 1 à 5, caractérisé en ce que, dans ce composé:
R(1) représente un atome d'hydrogène ou un groupe alkyle ayant 1, 2 ou 3 atomes de carbone, ou cycloalkyle ayant 3 atomes de carbone;
R(2) représente le groupe méthoxy ou éthoxy;
R(3) et R(4) identiques ou différents, représentent un atome d'hydrogène ou un groupe alkyle ayant 5, 6, 7 ou 8 atomes de carbone, cycloalkyle ayant 5 ou 6 atomes de carbone, ou un groupe (C₅₋₈H₁₁₋₁₇) dans lequel 1, 2, 3 ou 4 groupes CH₂ sont remplacés par des hétéroatomes O ou S ou par NH;
ou
R(3) et R(4) forment ensemble un groupe (CH₂)₅₋₈, dans lequel l'un des groupes CH₂ peut être remplacé par un hétéroatome O, S ou par NH;
E représente un atome d'oxygène ou de soufre;
X représente un atome d'oxygène;
Y représente un groupe [CR(5)R(5')]ₘ,
R(5) et R(5') représentant, indépendamment l'un de l'autre, un atome d'hydrogène ou le groupe méthyle, les chaînons [CR(5)R(5')] étant identiques ou différents;
m étant 1 ou 2;
et sels pharmaceutiquement acceptables d'un tel composé.

7. Composé de formule I selon au moins l'une des revendications 1 à 6, caractérisé en ce que, dans ce composé:
R(1) représente un atome d'hydrogène ou un groupe alkyle ayant 1, 2 ou 3 atomes de carbone, ou cycloalkyle ayant 3 atomes de carbone;
R(2) représente le groupe méthoxy ou éthoxy;
R(3) représente un atome d'hydrogène;
R(4) représente un groupe alkyle ayant 5, 6, 7 ou 8 atomes de carbone, cycloalkyle ayant 5 ou 6 atomes de carbone, ou un groupe (C₅₋₈H₁₁₋₁₇) dans lequel 1, 2, 3 ou 4 groupes CH₂ sont remplacés par des hétéroatomes O, S ou par NH;
E représente un atome d'oxygène ou de soufre;
X représente un atome d'oxygène;
Y représente un groupe [CR(5)R(5')]ₘ,
R(5) et R(5') représentant, indépendamment l'un de l'autre, un atome d'hydrogène ou le groupe méthyle,
les chaînons [CR(5)R(5')] étant identiques ou différents;
m étant 1 ou 2;
et sels pharmaceutiquement acceptables d'un tel composé.

8. Benzènesulfonylthio-urée substituée de formule 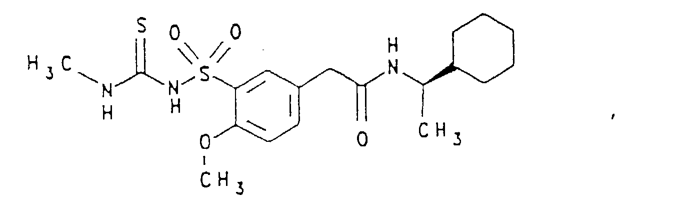 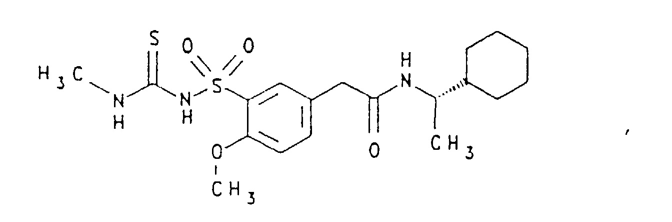 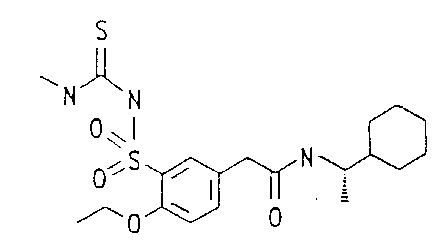 ou 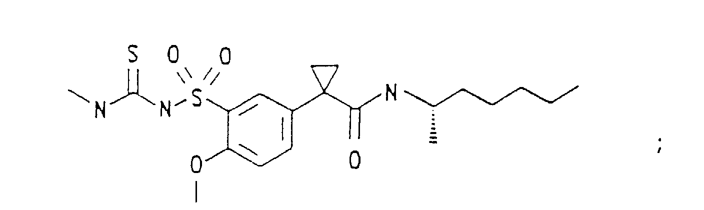 ainsi que ses sels pharmaceutiquement acceptables.

9. Procédé pour la préparation d'un composé de formule I selon la revendication 1, caractérisé en ce que
(a) on fait réagir un sulfonamide de formule II 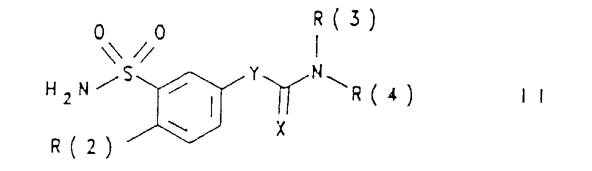 ou un sel de celui-ci, de formule III 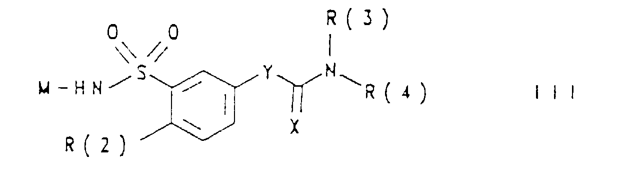 formules dans lesquelles R(2), R(3), R(4), X et Y ont les significations données dans la revendication 1, et le cation M représente un ion alcalin ou alcalino-terreux, avec un isocyanate substitué par R(1), de formule IV
R(1)-N=C=O IV
dans laquelle R(1) a la signification donnée dans la revendication 1,
pour aboutir à une benzènesulfonylurée substituée Ia; 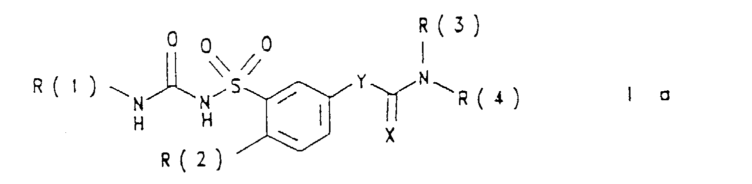 ou en ce que
(b) on prépare une benzènesulfonylurée de formule Ia à partir d'un benzènesulfonamide aromatique II ou d'un sel III de celui-ci, avec un trichloracétamide substitué par R(1), de formule V
Cl₃C-C(=O)-NH-R(1) V
en présence d'une base;
ou en ce que
(c) on prépare une benzènesulfonylthio-urée Ib 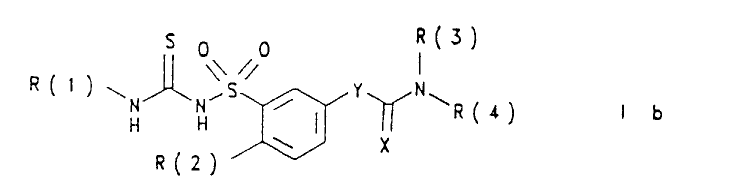 à partir d'un benzènesulfonamide II ou d'un de ses sels III et d'un thio-isocyanate VI substitué par R(1)
R(1)-N=C=S VI;
ou en ce que
(d) on prépare une benzènesulfonylurée de formule Ia par une réaction de conversion à partir d'une benzènesulfonylthio-urée de formule Ib; ou en ce que
(e) on prépare une benzènesulfonylurée Ia par la réaction d'une amine de formule R(1)-NH₂ avec un benzènesulfonyl-isocyanate de formule VII 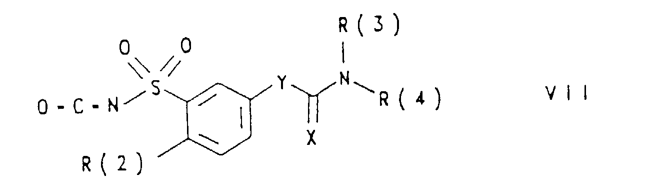 ou en ce que
(f) on prépare une benzènesulfonylthio-urée Ib par la réaction d'une amine de formule R(1)-NH₂ avec un benzène-sulfonylisothiocyanate de formule VIII 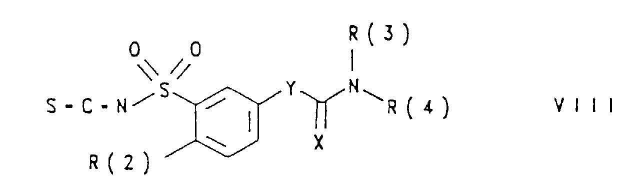 ou en ce que
(g) on prépare une benzènesulfonylurée Ia à partir d'une benzènesulfonylurée de formule IX 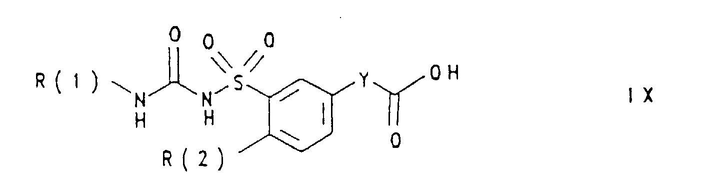 et R(3)R(4)NH, à l'aide d'agents de déshydratation ou par activation à l'aide d'halogénures de carbonyle ou formation d'anhydrides mixtes;
ou en ce que
(h) on prépare une benzènesulfonylthio-urée Ib à partir d'une benzènesulfonylthio-urée de formule X 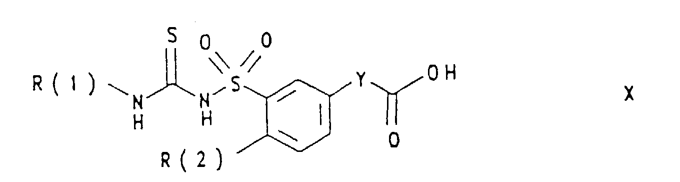 et R(3)R(4)NH, à l'aide d'agents de déshydratation ou par activation à l'aide d'halogénures de carbonyle ou formation d'anhydrides mixtes.

10. Utilisation d'un composé de formule I selon une ou plusieurs des revendications 1 à 8, ou d'un sel pharmaceutiquement acceptable de celui-ci, pour la fabrication d'un médicament destiné au traitement de troubles du rythme cardiaque.

11. Utilisation d'un composé de formule I selon une ou plusieurs des revendications 1 à 8, ou d'un sel pharmaceutiquement acceptable de celui-ci, pour la fabrication d'un médicament destiné à empêcher la mort subite par arrêt cardiaque.

12. Utilisation d'un composé de formule I selon une ou plusieurs des revendications 1 à 8, ou d'un sel pharmaceutiquement acceptable de celui-ci, pour la fabrication d'un médicament destiné au traitement d'états ischémiques du coeur.

13. Utilisation d'un composé de formule I selon une ou plusieurs des revendications 1 à 8, ou d'un sel pharmaceutiquement acceptable de celui-ci, pour la fabrication d'un médicament destiné au traitement de l'insuffisance cardiaque.

14. Utilisation d'un composé de formule I selon une ou plusieurs des revendications 1 à 8, ou d'un sel pharmaceutiquement acceptable de celui-ci, pour la fabrication d'un médicament destiné à l'amélioration de la fonction cardiaque après une greffe du coeur.

15. Médicament, caractérisé par une quantité efficace d'un composé de formule I selon une ou plusieurs des revendications 1 à 8, ou d'un sel pharmaceutiquement acceptable d'un tel composé.
